# EUROPÄISCHE PATENTSCHRIFT

(11) **EP 2 205 609 B1**
(45) Veröffentlichungstag und Bekanntmachung des Hinweises auf die Patenterteilung: **29.03.2017**
(21) Anmeldenummer: 08840075.9
(22) Anmeldetag: 16.10.2008
(51) Int. Cl.: C07D 495/04, C07D 519/00, A61K 31/519, A61P 1/00, A61P 11/00, A61P 25/00

(54) **HETEROCYCLUS-SUBSTITUIERTE PIPERAZINO-DIHYDROTHIENOPYRIMIDINE**
HETEROCYCLE SUBSTITUTED PIPERAZINO DIHYDROTHIENO PYRIMIDINES
COMPOSÉS PIPÉRAZINO-DIHYDRO-THIÉNOPYRIMIDINE SUBSTITUÉES PAR UN HÉTÉROCYCLE

(30) Priorität: 19.10.2007 EP 07118911
(43) Veröffentlichungstag der Anmeldung: 14.07.2010
(73) Patentinhaber: Boehringer Ingelheim International GmbH, 55216 Ingelheim am Rhein (DE)
(72) Erfinder: POUZET, Pascale, 55216 Ingelheim Am Rhein (DE); ANDERSKEWITZ, Ralf, 55216 Ingelheim Am Rhein (DE); DOLLINGER, Horst, 55216 Ingelheim Am Rhein (DE); FIEGEN, Dennis, 55216 Ingelheim Am Rhein (DE); FOX, Thomas, 55216 Ingelheim Am Rhein (DE); GOEGGEL, Rolf, 55216 Ingelheim Am Rhein (DE); HOENKE, Christoph, 55216 Ingelheim Am Rhein (DE); MARTYRES, Domnic, 55216 Ingelheim Am Rhein (DE); NICKOLAUS, Peter, 55216 Ingelheim Am Rhein (DE); KLINDER, Klaus, 55216 Ingelheim Am Rhein (DE)
(74) Vertreter: Simon, Elke Anna Maria
(86) Internationale Anmeldenummer: PCT/EP2008/063983
(87) Internationale Veröffentlichungsnummer: WO 2009/050242

(56) Entgegenhaltungen:
- WO-A-2006/111549

## Beschreibung

Die Erfindung betrifft neue Dihydrothienopyrimidinsulfoxide der Formel **1**, sowie pharmakologisch verträgliche Salze, Diastereomere, Enantiomere, Racemate, Hydrate oder Solvate davon, 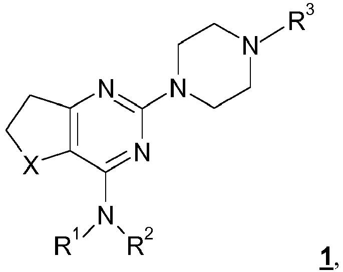
worin **X** SO ist und worin
R¹ H ist,
R² und R³ die in Anspruch 1 genannten Bedeutungen haben,
sowie pharmazeutische Zusammensetzungen, die diese Verbindungen beinhalten.

Diese neuen Dihydrothienopyrimidinsulfoxide sind geeignet zur Behandlung von Atemwegs- oder gastrointestinalen Beschwerden oder Erkrankungen, entzündlichen Erkrankungen der Gelenke, der Haut oder der Augen, Erkrankungen des periphären oder zentralen Nervensystems oder Krebserkrankungen.

### STAND DER TECHNIK

US 3,318,881 und BE 663693 offenbaren die Herstellung von Dihydrothieno[3,2-d]pyrimidine die kardiovaskulären und sedative Eigenschaften besitzen. WO 2006/111549 und EP06112779.1 (EP1847543) offenbaren jeweils Dihydrothienopyrimidinsulfoxide nach der obigen Formel **1,** wobei R³ jedoch ausschließlich für ein substituiertes Phenyl, nicht für einen Heterocyclus oder ein Heteroaryl, stehen kann.

### BESCHREIBUNG DER ERFINDUNG

Überraschenderweise konnte nun gefunden werden, dass Dihydrothienopyrimidinsulfoxide der Formel **1,** in denen R³ für einen mono- oder bicyclischen, ungesättigten, teilweise gesättigten oder gesättigten Heterocyclus oder für ein mono- oder bicyclisches Heteroaryl steht, insbesondere solche, in denen X SO bedeutet, besonders geeignet sind zur Behandlung entzündlicher Erkrankungen und gegenüber den entsprechenden Dihydrothienopyrimidinsulfoxiden aus dem Stand der Technik überlegen sind.

Gegenstand der vorliegenden Erfindung sind deshalb Verbindungen der Formel **A** 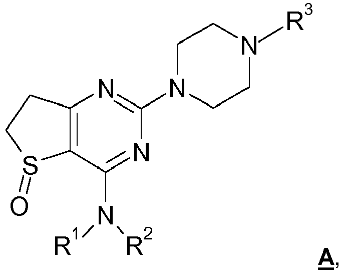 worin
- **R¹**: H,
- **R²**: ausgewählt ist aus der Gruppe bestehend aus 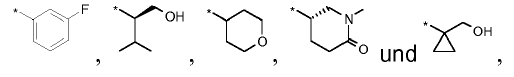
und worin
- **R³**: ein bicyclischer 9- bis 11-gliedriger Heterocyclus ausgewählt aus der Gruppe bestehend aus Benzoxazol, Benzodioxol, Dihydrobenzodioxin, Benzodioxin, Benzisoxazol, Benzothiazol, Benzisothiazol, Thienopyrimidin, Furopyrimidin, Thienopyridin, Furopyridin, Indol, Isoindol, Chinoxalin, Naphthyridin, Pyridopyrazin, Pyridopyrimidin, Chinolin, Isochinolin, Benzoimidazol, 6,7,8,9-Tetrahydro-5H-pyrazino[2,3-d]azepin, Benzothiophen, Benzofuran, Chinazolin, Indazol, Isobenzofuran und Pteridin ist,
der gegebenenfalls durch einen oder mehrere Reste ausgewählt aus der Gruppe bestehend aus
F, Cl, Br, CF₃, CHF₂, CH₂F, CN, OH, -Methyl, Ethyl, Propyl, Isopropyl, -O-Methyl und O-Ethyl, substituiert sein kann,
die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl, - COO(CH₃), -O-Methyl und -O-Ethyl substituiert sein können,
sowie pharmakologisch verträgliche Salze, Diastereomere, Enantiomere, Racemate, Hydrate oder Solvate davon.

Ein Gegenstand der vorliegenden Erfindung sind die Verbindungen der Formel **A** 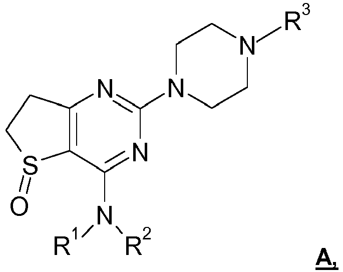 insbesondere aber sowohl die R-Enantiomere nach Formel **A'** als auch die S-Enantiomere nach Formel **A"** bezüglich des Stereozentrums am Sulfoxid-Schwefel-Atom, 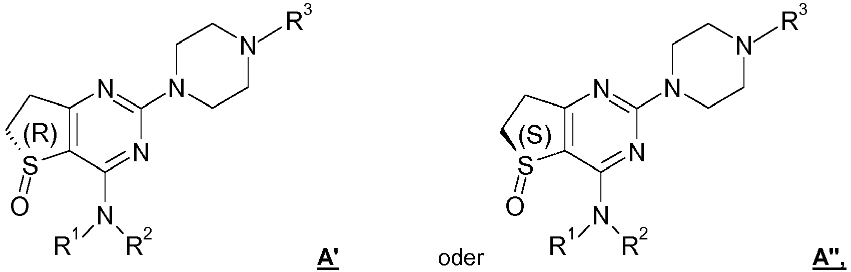 worin
- **R¹**: H ist,
- **R²**: ein Rest ausgewählt ist aus der Gruppe bestehend aus 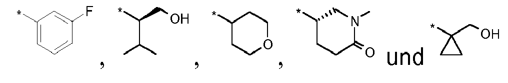 ist,
- **R³**: ein Rest ausgewählt ist aus der Gruppe bestehend aus 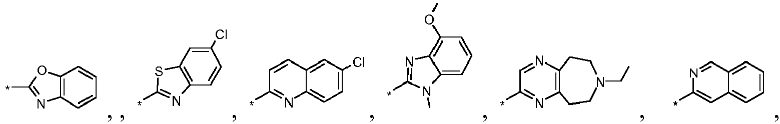 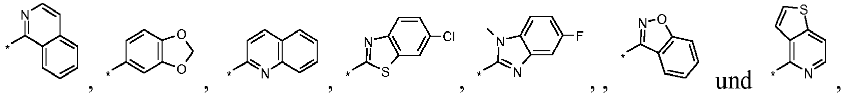
ist,
sowie pharmakologisch verträgliche Salze, Diastereomere, Enantiomere, Racemate, Hydrate oder Solvate davon.

Ein weiterer Gegenstand der Erfindung sind die obigen Verbindungen der Formel **A** als Arzneimittel.

Ein weiterer Gegenstand der Erfindung ist die Verwendung der obigen Verbindungen nach Formel **A** zur Herstellung eines Medikaments zur Behandlung von Krankheiten, die sich durch Inhibition des PDE4-Enzyms behandeln lassen.

Ein weiterer Gegenstand der Erfindung ist die Verwendung der obigen Verbindungen nach Formel **A** zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

Ein weiterer Gegenstand der Erfindung ist die Verwendung der obigen Verbindungen nach Formel **A** zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einhergehen.

Ein weiterer Gegenstand der Erfindung ist die Verwendung der obigen Verbindungen nach Formel **A** zur Herstellung eines Medikament zur Behandlung von entzündlichen und obstruktiven Erkrankungen wie COPD, chronischer Sinusitis, Asthma, Morbus Crohn, Colitis ulcerosa.

Ein weiterer Gegenstand der Erfindung ist die Verwendung der obigen Verbindungen nach Formel **A** zur Herstellung eines Medikaments zur Behandlung von entzündlichen Erkrankungen des Gastrointestinaltraktes.

Ein weiterer Gegenstand der Erfindung ist die Verwendung der obigen Verbindungen nach Formel **A** zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein weiterer Gegenstand der Erfindung sind pharmazeutische Formulierungen, die eine oder mehrere der obigen Verbindungen nach Formel **A** enthalten.

Ein weiterer Gegenstand der Erfindung sind pharmazeutische Formulierungen enthaltend eine oder mehrere Verbindungen der Formel **A** in Kombination mit einem oder mehreren Wirkstoffen ausgewählt aus der Gruppe bestehend aus Betamimetika, Corticosteroiden, weiteren PDE4-Inhibitoren, EGFR-Hemmern und LTD4-Antagonisten, CCR3-Inhibitoren, iNOS-Inhibitoren und SYK-Inhibitoren.

### VERWENDETE BEGRIFFE UND DEFINITIONEN

Soweit nicht anders angegeben, sind alle Substituenten voneinander unabhängig. Sollten an einer Gruppe beispielsweise mehrere C₁₋₆-Alkylgruppen als Substituenten möglich sein, so könnte, beispielsweise im Fall von drei Substituenten C₁₋₆-Alkyl unabhängig voneinander beispielsweise einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird ein Stern (*) in der Strukturformel des Substituenten als der Verknüpfungspunkt zum Rest des Moleküls verstanden. Weiterhin wird das auf den Verknüpfungspunkt folgende Atom des Substituenten als das Atom mit der Positionsnummer 1 verstanden. So werden zum Beispiel die Reste N-Piperidinyl (**I**), 4-Piperidinyl (**II**), 2-Tolyl (**III**), 3-Tolyl (**IV**) und 4-Tolyl (**V**) wie folgt dargestellt:

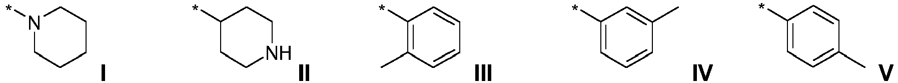

Befindet sich in der Strukturformel des Substituenten kein Stern (*), so kann an dem Substituenten jedes Wasserstoffatom entfernt werden und die dadurch frei werdende Valenz als Bindungsstelle zum Rest eines Molekül dienen, sofern der Anknüpfungspunkt zum Restmolekül nicht anderweitig bezeichnet oder definiert ist. So kann zum Beispiel **VI** die Bedeutung von 2-Tolyl, 3-Tolyl, 4-Tolyl und Benzyl haben.

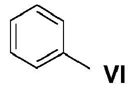

Unter dem Begriff "C₁₋₁₀-Alkyl" (auch soweit sie Bestandteil anderer Reste sind) werden verzweigte und unverzweigte Alkylgruppen mit 1 bis 10 Kohlenstoffatomen, unter dem Begriff "C₁₋₆-Alkyl" dementsprechend verzweigte und unverzweigte Alkylgruppen mit 1 bis 6 Kohlenstoffatomen verstanden. "C₁₋₄-Alkyl" steht entsprechend für verzweigte und unverzweigte Alkylgruppen mit 1 bis 4 Kohlenstoffatomen. Bevorzugt sind Alkylgruppen mit 1 bis 4 Kohlenstoffatomen. Beispielsweise werden hierfür genannt: Methyl, Ethyl, *n*-Propyl, *iso-*Propyl, *n*-Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen verstanden und unter dem Begriff "C₁₋₄-Alkylen" verzweigte und unverzweigte Alkylengruppen mit 1 bis 4 Kohlenstoffatomen verstanden. Bevorzugt sind Alkylengruppen mit 1 bis 4 Kohlenstoffatomen. Beispielsweise werden hierfür genannt: Methylen, Ethylen, Propylen, 1-Methylethylen, Butylen, 1-Methylpropylen, 1, 1-Dimethylethylen, 1, 2-Dimethylethylen, Pentylen, 1, 1-Dimethylpropylen, 2, 2, -Dimethylpropylen, 1, 2-Dimethylpropylen, 1, 3-Dimethylpropylen oder Hexylen. Sofern nicht anders beschrieben, umfassen die Definitionen Propylen, Butylen, Pentylen und Hexylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1, 1-Dimethylethylen, 1, 2-Dimethylethylen.

Sollte die Kohlenstoffkette mit einem Rest substituiert sein, der gemeinsam mit einem oder zwei Kohlenstoffatomen der Alkylenkette einen carbocyclischen Ring mit 3, 5 oder 6 Kohlenstoffatomen bildet, so sind damit unter anderem folgende Beispiele der Ringe umfasst:

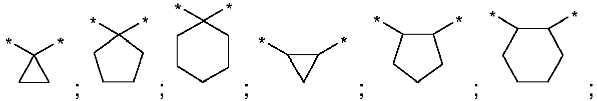

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkenylen" (auch soweit sie Bestandteil anderer Reste sind) werden verzweigte und unverzweigte Alkenylengruppen mit 2 bis 6 Kohlenstoffatomen verstanden und unter dem Begriff "C₂₋₄-Alkenylen" verzweigte und unverzweigte Alkylengruppen mit 2 bis 4 Kohlenstoffatomen verstanden. Bevorzugt sind Alkenylengruppen mit 2 bis 4 Kohlenstoffatome. Beispielsweise werden hierfür genannt: Ethenylen, Propenylen, 1-Methylethenylen, Butenylen, 1-Methylpropenylen, 1, 1-Dimethylethenylen, 1, 2-Dimethylethenylen, Pentenylen, 1, 1-Dimethylpropenylen, 2, 2, -Dimethylpropenylen, 1, 2-Dimethylpropenylen, 1, 3-Dimethylpropenylen oder Hexenylen. Sofern nicht anders beschrieben, umfassen die Definitionen Propenylen, Butenylen, Pentenylen und Hexenylen alle denkbaren isomeren Formen der jeweiligen Reste gleicher Kohlenstoffanzahl. So umfasst beispielsweise Propenyl auch 1-Methylethenylen und Butenylen umfasst 1-Methylpropenylen, 1, 1-Dimethylethenylen, 1, 2-Dimethylethenylen.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₂₋₆-Alkinylen" (auch soweit sie Bestandteil anderer Reste sind) werden verzweigte und unverzweigte Alkinylengruppen mit 2 bis 6 Kohlenstoffatomen verstanden und unter dem Begriff "C₂₋₄-Alkinylen" verzweigte und unverzweigte Alkylengruppen mit 2 bis 4 Kohlenstoffatomen verstanden. Bevorzugt sind Alkinylengruppen mit 2 bis 4 Kohlenstoffatome. Beispielsweise werden hierfür genannt: Ethinylen, Propinylen, 1-Methylethinylen, Butinylen, 1-Methylpropinylen, 1, 1-Dimethylethinylen, 1, 2-Dimethylethinylen, Pentinylen, 1, 1-Dimethylpropinylen, 2, 2, -Dimethylpropinylen, 1, 2-Dimethylpropinylen, 1, 3-Dimethylpropinylen oder Hexinylen. Sofern nicht anders beschrieben, umfassen die Definitionen Propinylen, Butinylen, Pentinylen und Hexinylen alle denkbaren isomeren Formen der jeweiligen Reste gleicher Kohlenstoffanzahl. So umfasst beispielsweise Propinyl auch 1-Methylethinylen und Butinylen umfasst 1-Methylpropinylen, 1, 1-Dimethylethinylen, 1, 2-Dimethylethinylen.

Unter dem Begriff "Aryl" (auch soweit sie Bestandteil anderer Reste sind) werden aromatische Ringsysteme mit 6 bis 10 Kohlenstoffatomen verstanden. Beispielsweise werden hierfür genannt: Phenyl oder Naphthyl, bevorzugter Arylrest ist Phenyl. Soweit nicht anders beschrieben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Aryl-C₁₋₆-alkylen" (auch soweit sie Bestandteil anderer Reste sind) werden verzweigte und unverzweigte Alkylengruppen mit 1 bis 6 Kohlenstoffatomen verstanden, die mit einem aromatischen Ringsystem mit 6 oder 10 Kohlenstoffatomen substituiert sind. Beispielsweise werden hierfür genannt: Benzyl, 1- oder 2-Phenylethyl oder 1- oder 2-Naphthylethyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heteroaryl-C₁₋₆-alkylen" (auch soweit sie Bestandteil anderer Reste sind) werden - obwohl auch bereits unter "Aryl-C₁₋₆-alkylen umfasst - verzweigte und unverzweigte Alkylengruppen mit 1 bis 6 Kohlenstoffatomen verstanden, die mit einem Heteroaryl substituiert sind.

Ein solches Heteroaryl umfasst fünf- oder sechsgliedrige heterocyclische Aromaten oder 5-10 gliedrige, bicyclische Heteroarylringe die ein, zwei oder drei Heteroatome, ausgewählt aus der Gruppe Sauerstoff, Schwefel und Stickstoff enthalten können und so viele konjugierte Doppelbindungen enthalten, dass ein aromatisches Systeme gebildet wird. Als Beispiele für fünf- oder sechsgliedrige heterocyclische Aromaten, werden genannt:

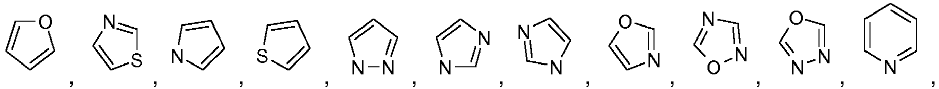

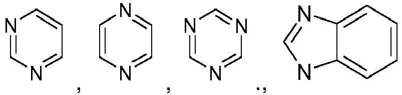

Soweit nicht anders beschreiben, können diese Heteroaryle substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Für die Heteroaryl-C₁₋₆-alkylene werden die folgenden Beispiele genannt:

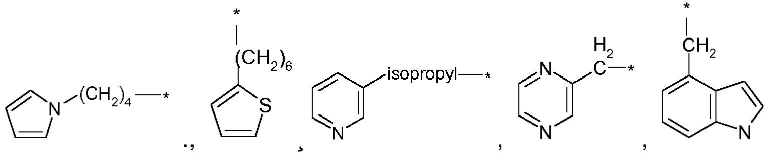

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatome. Beispielsweise werden hierfür genannt: CF₃, CHF₂, CH₂F, CH₂CF₃.

Unter dem Begriff "C₃₋₇-Cycloalkyl" (auch soweit sie Bestandteil anderer Reste sind) werden cyclische Alkylgruppen mit 3 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriffe "C₃₋₁₀-Cycloalkyl" werden zudem monocyclische Alkylgruppen mit 3 bis 7 Kohlenstoffatomen und auch bicyclische Alkylgruppen mit 7 bis 10 Kohlenstoffatomen verstanden oder auch monocyclische Alkylgruppen, die durch mindestens eine C₁₋₃-Kohlenstoffbrücke überbrückt sind.

Unter dem Begriff "heterocyclische Ringe" oder auch "Heterocyclus" werden fünf-, sechs- oder siebengliedrige, gesättigte oder ungesättigte heterocyclische Ringe verstanden die ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Obwohl unter dem Begriff "heterocyclische Ringe" oder "Heterocyclus" umfasst, definiert der Begriff "heterocyclische, nichtaromatische Ringe" fünf-, sechs- oder siebengliedrige ungesättigte Ringe. Als Beispiele werden genannt:

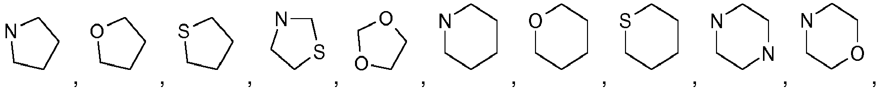

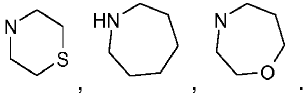

Obwohl unter dem Begriff "heterocyclische Ringe" oder "Heterocyclus" umfasst, definiert der Begriff "heterocyclische, aromatische Ringe" oder "Heteroaryl" fünf- oder sechsgliedrige heterocyclische Aromaten oder 5-10 gliedrige, bicyclische Heteroarylringe die ein, zwei, drei oder vier Heteroatome, ausgewählt aus der Gruppe Sauerstoff, Schwefel und Stickstoff, enthalten können und so viele konjugierte Doppelbindungen enthalten, dass ein aromatisches gebildet Systeme wird. Als Beispiele für fünf- oder sechsgliedrige heterocyclische Aromaten, werden genannt:

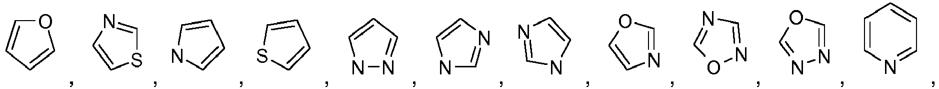

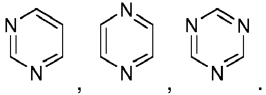

Soweit nicht anders erwähnt, kann ein heterocyclischer Ring (oder "Heterocyclus) mit einer Ketogruppe versehen sein. Als Beispiel hierfür werden genannt.

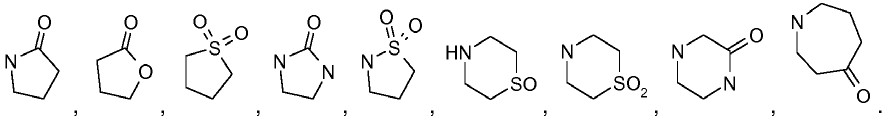

Obwohl unter "Cycloalkyl" bereits umfasst, werden unter dem Begriff "bicyclische Cycloalkyle" in der Regel acht-, neun- oder zehngliedrige, bicyclische Kohlenstoff-Ringe verstanden. Beispielhaft werden genannt:

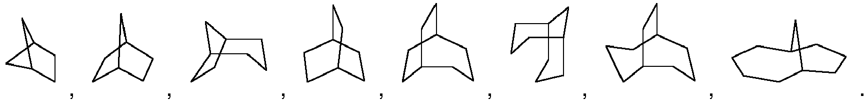

Obwohl unter "Heterocyclus" bereits umfasst, werden unter dem Begriff "bicyclische Heterocyclen" in der Regel acht-, neun- oder zehngliedrige, bicyclische Ringe verstanden, die ein oder mehrere Heteroatome, vorzugsweise 1-4, stärker bevorzugt, 1-3, noch stärker bevorzugt 1-2, insbesondere ein Heteroatom, ausgewählt aus der Gruppe Sauerstoff, Schwefel und Stickstoff enthalten können. Der Ring kann dabei über ein Kohlenstoffatom des Rings oder falls vorhanden über ein Stickstoffatom des Rings mit dem Molekül verknüpft sein. Beispielhaft werden genannt,

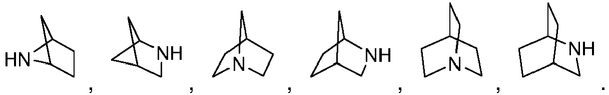

Obwohl unter "Aryl" bereits umfasst, wird unter einem "bicyclischen Aryl" ein 5-10 gliedriger, bicyclischer Arylring verstanden, der so viele konjugierte Doppelbindungen enthält, dass ein aromatisches System gebildet wird. Ein Beispiel für ein bicyclisches Aryl ist Naphthyl.

Obwohl unter "Heteroaryl" bereits umfasst, wird unter einem "bicyclischen Heteroaryl" ein 5-10 gliedriger, bicyclischer Heteroarylring verstanden, der ein, zwei, drei oder vier Heteroatome, ausgewählt aus der Gruppe Sauerstoff, Schwefel und Stickstoff, enthalten kann und so viele konjugierte Doppelbindungen enthält, dass ein aromatisches System gebildet wird.

Obwohl unter dem Begriff "bicyclische Cycloalkyle" oder "bicyclisches Aryl" umfasst, definiert der Begriff "kondensiertes Cycloalkyl" oder "kondensiertes Aryl" bicyclische Ringe, in denen die die Ringe trennende Brücke eine direkte Einfachbindung bedeutet. Als Beispiel für einen kondensiertes, bicyclisches Cycloalkyl werden genannt:

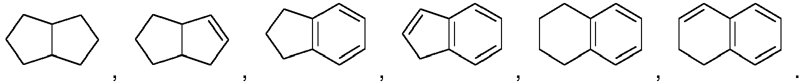

Obwohl unter dem Begriff "bicyclische Heterocyclen" oder "bicyclische Heteroaryle" umfasst, definiert der Begriff "kondensierte, bicyclische Heterocyclen " oder "kondensierte, bicyclische Heteroaryle"bicyclische 5-10 gliedrige Heteroringe die ein, zwei oder drei Heteroatome, ausgewählt aus der Gruppe Sauerstoff, Schwefel und Stickstoff enthalten und in denen die die Ringe trennende Brücke eine direkte Einfachbindung bedeutet. Die "kondensierten, bicyclischen Heteroaryle" enthalten zudem so viele konjugierte Doppelbindungen, dass ein aromatisches System gebildet wird. Beispielhaft werden genannt Pyrrolizin, Indol, Indolizin, Isoindol, Indazol, Purin, Chinolin, Isochinolin, Benzimidazol, Benzofuran, Benzopyran, Benzothiazol, Benzothiazol, Benzoisothiazol, Pyridopyrimidin, Pteridin, Pyrimidopyrimidin,

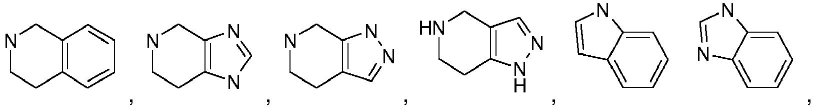

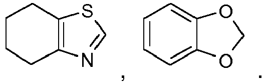

Unter dem Begriff "heterocyclische Spiroringe" (Spiro) werden 5-10 gliedrige, spirocyclische Ringe verstanden die gegebenenfalls ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Soweit nicht anders erwähnt, kann ein spirocyclischer Ring mit einer Oxo-, Methyl- oder Ethylgruppe versehen sein. Als Beispiele hierfür werden genannt:

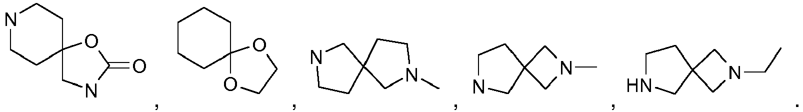

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel **1** können Säuregruppen besitzen, hauptsächlich Carboxylgruppen, und/oder basische Gruppen wie z.B. Aminofunktionen. Verbindungen der allgemeinen Formel **A** können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Wie vorstehend genannt, können die Verbindungen der Formel **A** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **A** mit anorganischen oder organischen Säuren vorliegen. Andererseits kann die Verbindung der Formel **A** im Falle von R gleich Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel A in der R Wasserstoff bedeutet, kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel **A** in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Die erfindungsgemäßen Verbindungen können gegebenenfalls als Racemate vorliegen, sie können aber auch als reine Enantiomere, d.h. in (R)- oder (S)-Form gewonnen werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Gegenstand der Erfindung sind die jeweiligen Verbindungen der Formel **A** in Form ihrer pharmakologisch verträglichen Salze wie vorstehend beschrieben. Diese pharmakologisch verträglichen Salze der Verbindungen der Formel **A** können ebenfalls in Form ihrer jeweiligen Hydrate (z.B. Monohydrate, Dihydrate etc.) sowie in Form Ihrer jeweiligen Solvate vorliegen.

Unter einem Hydrat der Verbindung nach Formel **A** versteht man im Rahmen der Erfindung ein kristallines, kristallwasserhaltiges Salz der Verbindung nach Formel **1.**

Unter einem Solvat der Verbindung nach Formel **A** versteht man im Rahmen der Erfindung ein kristallines Salz der Verbindung nach Formel **A,** welches Lösungsmittelmoleküle-Moleküle (z.B. Ethanol, Methanol etc) im Kristallgitter enthalten.

Dem Fachmann sind Standardverfahren zur Gewinnung von Hydraten und Solvaten (z.B. das Umkristallisieren aus dem entsprechenden Lösungsmittel bei Solvaten bzw. aus Wasser bei Hydraten) bekannt.

### SYNTHESEVORSCHRIFTEN

Die Verbindungen der allgemeinen Formel **A** können nach folgendem allgemeinen Syntheseschema hergestellt werden, wobei die Substituenten der allgemeinen Formel **A** die zuvor genannten Bedeutungen haben. Diese Verfahren sind als Erläuterung der Erfindung zu verstehen ohne selbige auf deren Gegenstand zu beschränken.

### ALLGEMEINE SYNTHESE SCHEMA

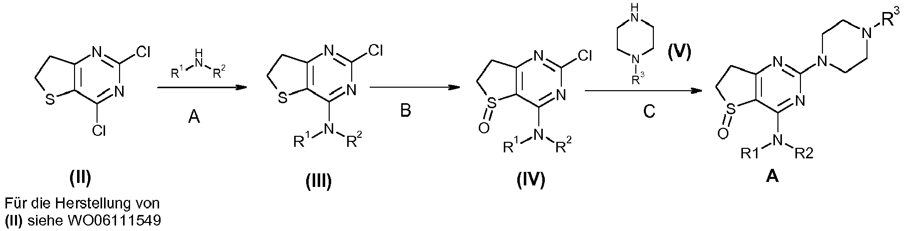

### 1. SYNTHESE VON (3-FLUORPHENYL)-[5-OXO-2-(4-THIAZOL-2-YL-PIPERAZIN-1-YL)-6,7-DIHYDRO-5H-5λ⁴-THIENO[3,2-d]PYRIMIDIN-4-YL]-AMIN, (BEISPIEL 1)

### 1.1 (2-Chlor-6,7-dihydrothieno[3,2-d]pyrimidin-4-yl)-(3-fluorphenyl)-amin (III-1)

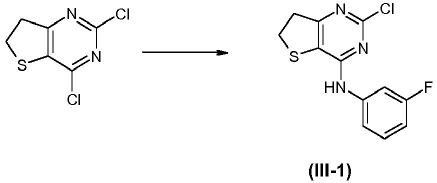

4 g **(II)** werden in 15 ml Dimethylformamid vorgelegt, anschliessend werden 4,5 ml Diisopropylethylamin und dann 2,5 ml 3-Fluorphenylamin zugegeben. Das Reaktionsgemisch wird bei 120°C, bis keine weitere Umsetzung erfolgt, erhitzt und nach Abkühlen eingedampft. Der Rückstand wird mit Wasser versetzt. Das Produkt wird mit Dichlormethan extrahiert und chromatographisch (Kieselgel, Petrolether/Essigester 80/20 bis 60/40) gereinigt. 2,6 g **(III-1)** werden als Feststoff erhalten. Analytische HPLC (Methode A): RT = 3,27 min.

### 1.2 2-Chlor-5-oxo-6,7-dihydro-5H-5λ⁴-thieno[3,2-d]pyrimidin-4-yl)-(3-fluorphenyl)-amin (IV-1)

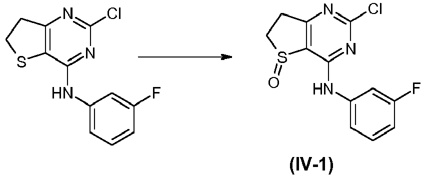

0,102 g S-(-)-1,1'-Bi-2-naphtol werden in 0,5 ml Chloroform unter Argon vorgelegt, dann 0,052 ml Titan(IV)-isopropylat und 0,064 ml Wasser zugegeben. Das Reaktionsgemisch wird 45 Minuten bei Raumtemperatur gerührt. Anschließend wird eine Suspension von 0,5 g **(III-1)** in 25 ml Chloroform zugegeben. Das Reaktionsgemisch wird auf-2°/-4°C abgekühlt und nach 20 Minuten werden 0,323 ml tert-Butylhydroperoxid 5-6 M in Decan zugetropft. Das Reaktionsgemisch wird bei -2/-4°C, bis keine weitere Umsetzung erfolgt, weitergerührt und mit Wasser versetzt. Das Produkt wird mit Dichlormethan extrahiert und chromatographisch (Kieselgel, Dichlormethan/Methanol 100/0 bis 95/5) gereinigt. 0,47 g **(IV-1)** werden als Feststoff erhalten. Analytische HPLC-MS (Methode A): RT = 1,16 min.

### 1.3 (3-Fluorphenyl)-[5-oxo-2-(4-thiazol-2-yl-piperazin-1-yl)-6,7-dihydro-5H-5λ⁴-thieno[3,2-d]pyrimidin-4-yl]-amin (Beispiel 1)

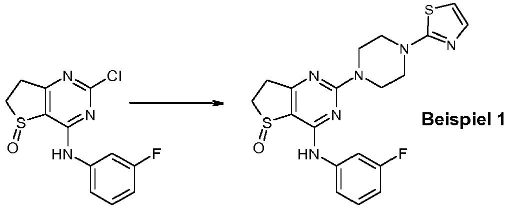

0,2 g **(IV-1)** wird in 3 ml Dioxan vorgelegt, 240 µl Diisopropylethylamin und 0,24 g 1-Thiazol-2-yl-piperazin werden zugefügt. Das Reaktionsgemisch wird bei 120°C, bis keine weitere Umsetzung erfolgt, in der Mikrowelle erhitzt und mit Wasser versetzt. Der ausgefallene Feststoff wird abgesaugt und chromatographisch (Kieselgel, Essigester/Methanol 100/0 bis 80/20) gereinigt. 0,17 g **Beispiel 1** werden als Feststoff erhalten. Analytische HPLC-MS (Methode A): RT = 1,07 min.

### 2. SYNTHESE VON (R)-3-METHYL-2-[5-OXO-2-(4-THIAZOL-2-YL-PIPERAZIN-1-YL)-6,7-DIHYDRO-5H-5λ⁴-THIENO[3,2-d]PYRIMIDIN-4-YLAMINO]-BUTAN-1-OL (BEISPIEL 2)

### 2.1 (R)-2-(2-Chlor-6,7-dihydro-thieno[3,2-d]pyrimidin-4-ylamino)-3-methyl-butan-1-ol (III-2):

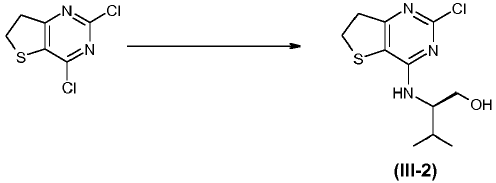

7,2 g 2,4-Dichlor-6,7-dihydro-thieno[3,2-*d*]pyrimidin **(II)** werden in 36 ml Dioxan vorgelegt, anschliessend werden 18 ml Diisopropylethylamin und dann 6,1 g (R)-(-)-2-Amino-3-Methyl-1-butanol zugegeben. Das Reaktionsgemisch wird bei 100°C, bis keine weitere Umsetzung erfolgt, erhitzt und nach Abkühlen eingedampft. Der Rückstand wird mit Petrolether/Essigester 9:1 im Ultraschallbad behandelt und der Feststoff abgesaugt und getrocknet. 8,3 g **(III-2)** werden als Feststoff erhalten. Analytische HPLC (Methode A): RT = 2,75 min

### 2.2 (R)-2-(2-Chlor-5-oxo-6,7-dihydro-5H-5λ⁴-thieno[3,2-d]pyrimidin-4-ylamino)-3-methyl-butan-1-ol (IV-2) :

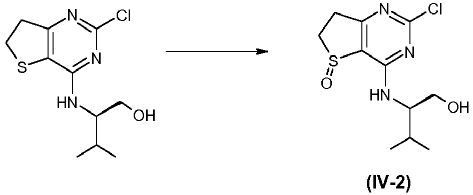

4,1 g S-(-)-1,1'-Bi-2-naphtol werden in 15 ml Chloroform unter Argon vorgelegt, dann 0,44 ml Titan(IV)-isopropylat und 0,54 ml Wasser zugegeben. Das Reaktionsgemisch wird 1 Stunde bei Raumtemperatur gerührt. Anschließend wird eine Suspension von 4,1 g **(III-2)** in 107 ml Dichlormethan zugegeben. Das Reaktionsgemisch wird auf -2°C abgekühlt und nach 30 Minuten werden 2,7 ml tert-Butylhydroperoxid 5-6 M in Decan zugetropft. Das Reaktionsgemisch wird bei -2 °C, bis keine weitere Umsetzung erfolgt, weitergerührt und mit NH₄OH basisch gestellt. Das Produkt wird mit Dichlormethan extrahiert und chromatographisch (Kieselgel, Essigester/Methanol 100/0 bis 86/14) gereinigt. 2,45 g **(IV-2)** werden als Feststoff erhalten. Analytische HPLC-MS (Methode A): RT = 0,98 min

### 2.3 (R)-3-Methyl-2-[5-oxo-2-(4-thiazol-2-yl-piperazin-1-yl)-6,7-dihydro-5H-5λ⁴-thieno[3,2-d]pyrimidin-4-ylamino]-butan-1-ol (Beispiel 2)

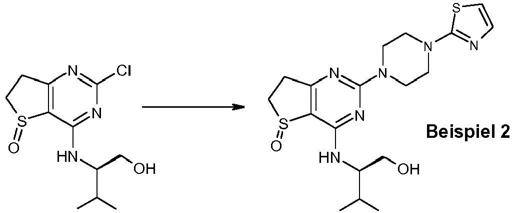

Ausgehend von 0,2 g **(IV-2)** (siehe 2.2) und 0,245 g 1-Thiazol-2-yl-piperazin werden 0,13 g **Beispiel 2** analog zu Beispiel 1 (siehe 1.3) hergestellt. Das Reaktionsgemisch wird mit Wasser versetzt und das Produkt mit Dichlormethan extrahiert und chromatographisch (Kieselgel, Dichlormethan/Methanol 100/0 bis 90/10) gereinigt. Analytische HPLC-MS (Methode A): RT = 0,87 min.

### 3. SYNTHESE VON [2-(4-BENZOXAZOL-2-YL-PIPERAZIN-1-YL)-5-OXO-6,7-DIHYDRO-5H-5λ□⁴-THIENO[3,2-d]PYRIMIDIN-4-YL]-(3-FLUORPHENYL)-AMIN (BEISPIEL 3)

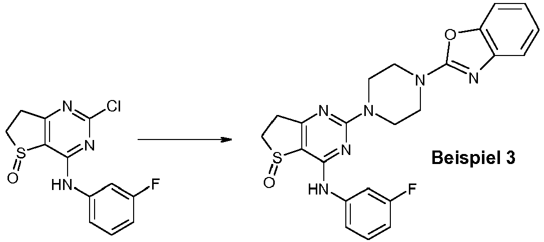

Ausgehend von 0,2 g **(IV-1)** (siehe 1.2) und 0,287 g 2-Piperazin-1-yl-benzoxazol werden 0,31 g **Beispiel 3** analog zu Beispiel 1 (siehe 1.3) hergestellt. Das Reaktionsgemisch wird mit Wasser versetzt und das Produkt abgesaugt. Analytische HPLC-MS (Methode A): RT = 1,23 min.

### 4. SYNTHESE VON [2-(4-BENZOXAZOL-2-YL-PIPERAZIN-1-YL)-5-OXO-6,7-DIHYDRO-5H-5λ⁴-THIENO[3,2-d]PYRIMIDIN-4-YL]-(TETRAHYDROPYRAN-4-YL)-AMINE (BEISPIEL 5)

### 4.1 (2-Chlor-6,7-dihydrothieno[3,2-d]pyrimidin-4-yl)-(tetrahydropyran-4-yl)-amin (III-3):

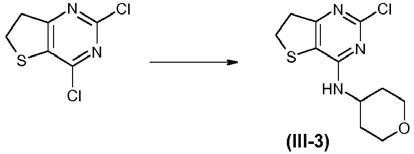

0,68 g **(II)** werden in 6 ml Dioxan vorgelegt, anschliessend werden 1,72 ml Diisopropylethylamin und dann 0,6 g 4-Aminotetrahydropyran zugegeben. Das Reaktionsgemisch wird bei 130°C, bis keine weitere Umsetzung erfolgt, erhitzt und nach Abkühlen eingedampft. Das Produkt wird mit Wasser im Ultraschallbad behandelt und der Feststoff abgesaugt und getrocknet. 0,66 g **(III-3)** werden erhalten. Analytische HPLC-MS (Methode A): RT = 1,08 min.

### 4.2 (2-Chlor-5-oxo-6,7-dihydro-5H-5λ⁴-thieno[3,2-d]pyrimidin-4-yl)-(tetrahydropyran-4-yl)-amin (IV-3):

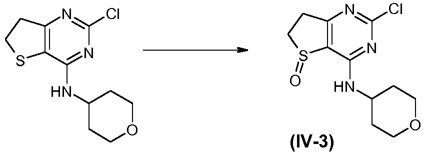

0,14 g S-(-)-1,1'-Bi-2-naphtol werden in 5 ml Chloroform unter Argon vorgelegt, dann 0,072 ml Titan(IV)-isopropylat und 0,087 ml Wasser zugegeben. Das Reaktionsgemisch wird 45 Minuten bei Raumtemperatur gerührt. Anschließend wird eine Suspension von 0,66 g **(III-3)** in 25 ml Chloroform zugegeben. Das Reaktionsgemisch wird auf -10°C abgekühlt und nach 60 Minuten werden 0,444 ml tert-Butylhydroperoxid 5-6 M in Decan zugetropft. Das Reaktionsgemisch wird bei -10 bis -4°C, bis keine weitere Umsetzung erfolgt, weitergerührt und mit Wasser versetzt. Das Produkt wird mit Dichlormethan extrahiert und chromatographisch (Kieselgel, Essigester/Methanol 100/0 bis 80/20) gereinigt. 0,42 g **(IV-3)** werden als Feststoff erhalten. Analytische HPLC-MS (Methode A): RT = 0,94 min.

### 4.3 [2-(4-Benzoxazol-2-yl-piperazin-1-yl)-5-oxo-6,7-dihydro-5H-5λ⁴-thieno[3,2-d]pyrimidin-4-yl]-(tetrahydropyran-4-yl)-amin (Beispiel 5)

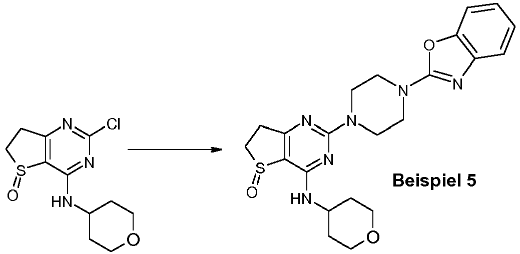

Ausgehend von 0,2 g **(IV-3)** (siehe 4.2) und 0,315 g 2-Piperazin-1-yl-benzoxazol werden 0,3 g **Beispiel 5** analog zu **Beispiel 3** (siehe 3.) hergestellt und aufgearbeitet. Analytische HPLC-MS (Methode A): RT = 1,04 min.

### 5. SYNTHESE VON (R)-2-{2-[4-(6-CHLORPYRIDAZIN-3-YL)-PIPERAZIN-1-YL]-5-OXO-6,7-DIHYDRO-5H-5λ⁴-THIENO[3,2-d]PYRIMIDIN-4-YLAMINO}-3-METHYLBUTAN-1-OL (BEISPIEL 6)

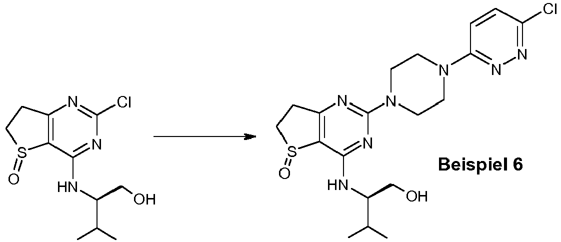

Ausgehend von 0,2 g **(IV-2)** (siehe 2.2) und 0,287 g 3-Chlor-6-piperazin-1-yl-pyridazin, werden 0,257g **Beispiel 6** analog zu Beispiel 3 (siehe 3.) hergestellt und aufgearbeitet. Analytische HPLC-MS (Methode A): RT = 0,98 min.

### 6. SYNTHESE VON {2-[4-(6-CHLORPYRIDAZIN-3-YL)-PIPERAZIN-1-YL]-5-OXO-6,7-DIHYDRO-5H-5λ⁴-THIENO[3,2-d]PYRIMIDIN-4-YL}-(3-FLUORPHENYL)-AMIN (BEISPIEL 7)

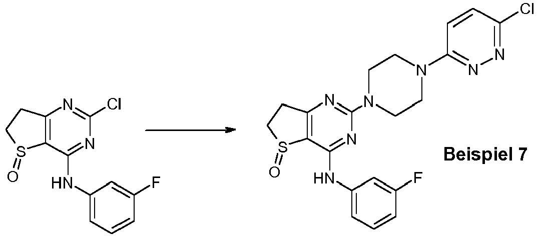

Ausgehend von 0,2 g **(IV-1)** (siehe 1.2) und 0,28 g 3-Chlor-6-piperazin-1-yl-pyridazin, werden 0,31 g **Beispiel 7** analog zu **Beispiel 3** (siehe 3.) hergestellt. Analytische HPLC-MS (Methode A): RT = 1,12 min.

### 7. SYNTHESE VON (R)-2-[2-(4-BENZOXAZOL-2-YL-PIPERAZIN-1-YL)-5-OXO-6,7-DIHYDRO-5H-5λ⁴-THIENO[3,2-d]PYRIMIDIN-4-YLAMINO]-3-METHYLBUTAN-1-OL (BEISPIEL 10)

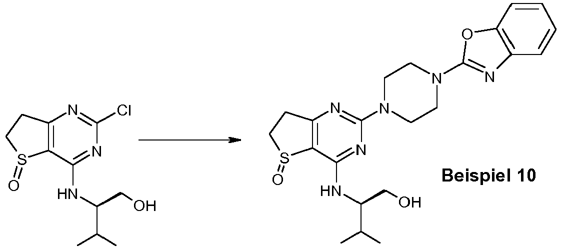

Ausgehend von 0,2 g **(IV-2)** (siehe 2.2) und 0,313 g 2-Piperazin-1-yl-benzoxazol werden 0,16 g **Beispiel 10** analog zu Beispiel 1 (siehe 1.3) hergestellt. Das Reaktionsgemisch wird mit Wasser versetzt und das Produkt mit Dichlormethan extrahiert und chromatographisch ( Kieselgel, Essigester/Methanol 100/0 bis 80/20) gereinigt.. Analytische HPLC-MS (Methode A): RT = 1,06 min.

### 8. SYNTHESE VON (1-{2-[4-(5-CHLORPYRIDIN-2-YL)-PIPERAZIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 13)

### 8.1 (1-Hydroxymethylcyclopropyl)-carbamidsäure tert-butylester:

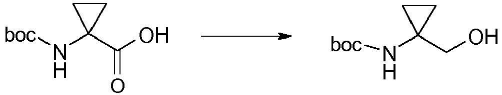

1 g 1-(BOC-amino)-cyclopropancarbonsäure wird in 20 ml Dimethoxyethan gelöst und auf-70°C abgekühlt. Dann werden 0,65 ml N-Methylmorpholin zugegeben und 0,71 ml Isobutylchloroformiat in 5 ml Dimethoxyethan zugetropft. Das Reaktionsgemisch wird auf-5°C erwärmt. Der Niederschlag wird abgesaugt. Das Eluat wird auf -15°C abgekühlt und 0,303 g Natriumborhydrid werden langsam zugegeben. Das Reaktionsgemisch wird anschliessend 30 Minuten bei Raumtemperatur gerührt, mit Wasser versetzt und das Produkt mit Dichlormethan extrahiert. Die organische Phase wird getrocknet und zur Trockne eingedampft. 1,04 g Produkt werden als Feststoff erhalten. ¹H NMR (400 MHz, DMSO): 1,36 (9H, s); 0,61 (2H, t); 0,52 (2H, t).

### 8.2 1-Aminocyclopropanmethanol:

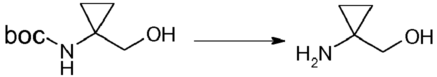

1,04 g (1-Hydroxymethylcyclopropyl)-carbamidsäure *tert*-butylester werden in 5 ml Dioxan vorgelegt. 2,5 ml HCl in Dioxan (4 mol/l) werden zugetropft. Das Reaktionsgemisch wird bei Raumtemperatur 15 h gerührt. Das Lösungsmittel wird zur Hälfte eingedampft und der ausgefallene Feststoff abgesaugt. 0,5 g Produkt werden als Hydrochlorid erhalten. ¹H NMR (400 MHz, DMSO): 5,27 (1 H, t); 0,91 (2H, t); 0,71 (2H, t).

### 8.3 [1-(2-Chlor-6,7-dihydrothieno[3,2-d]pyrimidin-4-ylamino)-cyclopropyl]-methanol (III-4) :

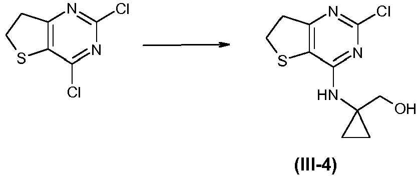

1,4 g **(II)** werden in 10 ml Dioxan vorgelegt, erst werden 3,6 ml Diisopropylethylamin, dann 1 g 1-Aminocyclopropanmethanol (siehe 8.2) zugegeben. Das Reaktionsgemisch wird bei 160°C, bis keine weitere Umsetzung erfolgt, erhitzt und nach Abkühlen eingedampft. Der Rückstand wird mit Cyclohexan/Essigester (8:2) im Ultraschallbad behandelt und der Feststoff abgesaugt und getrocknet. 1,24 g **(III-4)** werden als Feststoff erhalten. Analytische HPLC-MS (Methode A): RT = 1,01 min.

### 8.4 [1-(2-Chlor-5-oxo-6,7-dihydro-5H-5λ⁴-thieno[3,2-d]pyrimidin-4-ylamino)-cyclopropyl]-methanol (IV-4) :

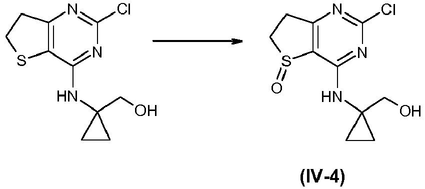

0,28 g S-(-)-1,1'-Bi-2-naphtol werden in 20 ml Chloroform unter Argon vorgelegt, dann werden 0,14 ml Titan(IV)-isopropylat und 0,17 ml Wasser zugegeben. Das Reaktionsgemisch wird 1 Stunde bei Raumtemperatur gerührt. Anschließend wird eine Suspension von 1,2 g **(III-4)** in 40 ml Dichlormethan und 2 ml Methanol zugegeben. Das Reaktionsgemisch wird auf -5°C abgekühlt und nach 30 Minuten werden 0,91 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Die Wasserphase wird wird mit Dichlormethan gewaschen und gefriergetrocknet. 1 g **(IV-4)** wird als Feststoff erhalten. Analytische HPLC-MS (Methode A) RT = 0,85 min.

### 8.5 (1-{2-[4-(5-Chlorpyridin-2-yl)-piperazin-1-yl]-5-oxo-6,7-dihydro-5H-5λ⁴-thieno[3,2-d]pyrimidin-4-ylamino}-cyclopropyl)-methanol (Beispiel 13)

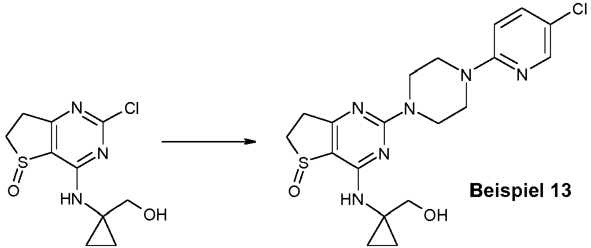

0,1 g **(IV-4)** (siehe 8.4) wird in 3 ml N-Methyl-2-pyrrolidon vorgelegt, anschliessend werden 182 µl Diisopropylethylamin und 0,08 g 1-(5-Chlorpyridin-2-yl)-piperazin zugefügt. Das Reaktionsgemisch wird bei 120°C, bis keine weitere Umsetzung erfolgt, in der Mikrowelle erhitzt. Das Produkt wird chromatographisch (präparative HPLC, Methode A) gereinigt. Analytische HPLC-MS (Methode B): RT = 1,09 min.

### 9. SYNTHESE VON {2-[4-(5-CHLORPYRIDIN-2-YL)-PIPERAZIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 14)

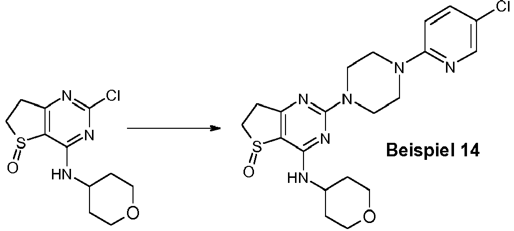

Ausgehend von 0,11 g **(IV-3)** (siehe 4.2) und 0,083 g 1-(5-Chlorpyridin-2-yl)-piperazin werden 0,14 g **Beispiel 14** analog zu Beispiel 13 (siehe 8.5) hergestellt und aufgereinigt. Analytische HPLC-MS (Methode B): RT = 1,14 min.

### 10. SYNTHESE VON {2-[4-(3-DIMETHYLAMINOPYRIDAZIN-4-YL)-PIPERAZIN-1-YL]-5-OXO-6,7-DIHYDRO-5H-5λ⁴-THIENO[3,2-D]PYRIMIDIN-4-YL}-(3-FLUORPHENYL)-AMIN TRIFLUORACETAT (BEISPIEL 15)

### 10.1 3,4,6-Trichlorpyridazin

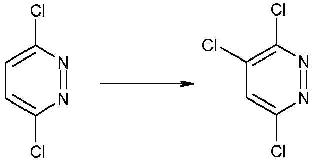

44 g 3,6-Dichlorpyridazin und 22 g Aluminiumtrichlorid werden auf 140°C erwärmt. Bei dieser Temperatur werden 10,6 I Chlor über 4 Stunden in das Reaktionsgemisch eingeleitet. Nach dem Abkühlen wird das Produkt mit Toluol extrahiert, mit einer 10%igen Natriumchlorid Lösung gewaschen und destilliert (Kp = 127 - 129°C). Es werden 44,1 g Produkt erhalten.

### 10.2 3,6-Dichlor-4-piperazin-1-yl-pyridazin

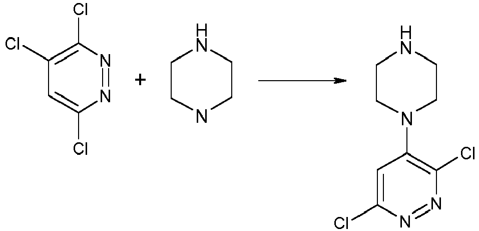

18 g 3,4,6-Trichlor-pyridazin und 34 g Piperazin werden in 100 ml Ethanol suspendiert und bei Raumtemperatur 30 Minuten gerührt. Der ausgefallene Feststoff wird abgesaugt. 500 ml Wasser werden zu der Mutterlauge zugegeben und das ausgefallene Produkt wird abgesaugt. 14 g Produkt werden als Feststoff erhalten. Smp = 111-115°C.

### 10.3 (6-Chlor-4-piperazin-1-yl-pyridazin-3-yl)-dimethylamin

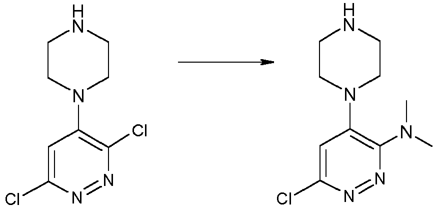

23 g 3,6-Dichlor-4-piperazin-1-yl-pyridazin und 45 g Dimethylamin werden in 200 ml Methanol suspendiert und 4 Stunden bei 100°C autoklaviert. Das Reaktionsgemisch wird zur Trockne eingedampft und das Produkt mit Chloroform extrahiert und mit Natronlauge gewaschen. Das Hydrochlorid wird mit einer etherischen HCl Lösung gefällt. 27 g Produkt werden erhalten. Smp = 291 °C.

### 10.4 Dimethyl-(4-piperazin-1-yl-pyridazin-3-yl)-amin (V-1)

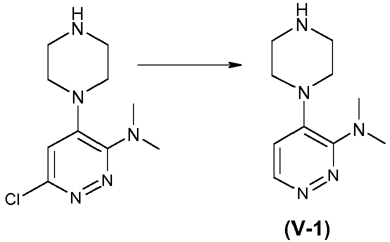

9,4 g (6-Chlor-4-piperazin-1-yl-pyridazin-3-yl)-dimethylamin Hydrochlorid und 7,3 g Natriumacetat werden in 150 ml Methanol suspendiert und mit 1 g Pd/C 10% bei Raumtemperatur hydriert. Der Katalysator wird abgesaugt, das Filtrat zur Trockne eingedampft und das Produkt mit Chloroform extrahiert und mit Natronlauge gewaschen. Das Hydrochlorid wird mit einer etherischen HCl Lösung gefällt. 7 g **(V-1)** werden erhalten. Smp = 335°C.

### 10.5 {2-[4-(3-Dimethylaminopyridazin-4-yl)-piperazin-1-yl]-5-oxo-6,7-dihydro-5H-5λ⁴-thieno[3,2-d]pyrimidin-4-yl}-(3-fluorphenyl)-amin (Beispiel 15)

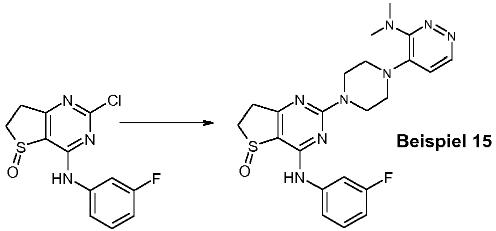

**(IV-1)** (siehe 1.2) (0,1 mmol) wird in 750 µl N-Methyl-2-pyrrolidon (NMP) und 50 µl Diisopropylethylamin vorgelegt, mit einer Lösung von Dimethyl-(4-piperazin-1-yl-pyridazin-3-yl)-amin (0,1 mmol, siehe 10.4) in 400µl NMP versetzt und 30 min bei 120°C in der Mikrowelle erhitzt. Anschliessend werden 600 µL DMF zugesetzt, die Reaktionslösung wird chromatographisch (präparative HPLC-MS, Methode A) aufgereinigt und die Produktfraktionen gefriergetrocknet. **Beispiel 15** wird als Trifluoracetat erhalten. Analytische HPLC-MS (Methode C): RT = 1,61 min.

### 11. SYNTHESE VON 6-CHLOR-4-{4-[4-(3-FLUORPHENYLAMINO)-5-OXO-6,7-DIHYDRO-5H-5λ⁴-THIENO[3,2-d]PYRIMIDIN-2-YL]-PIPERAZIN-1-YL}-PYRIDAZIN-3-OL (BEISPIEL 16)

### 11.1 (6-Chlor-4-piperazin-1-yl-pyridazin-3-yloxy)-ethanol

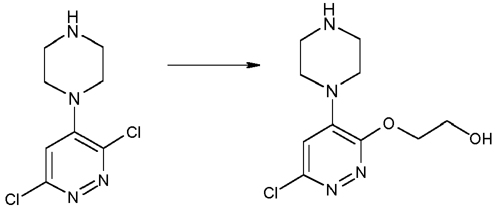

23 g 3,6-Dichlor-4-piperazin-1-yl-pyridazin (siehe 10.2) werden in 100 ml Ethylenglycol suspendiert und zu einer Suspension von 2,3 g Natrium in 100 ml Ethylenglycol getropft. Das Reaktionsgemisch wird 3 Stunden bei 100°C erhitzt und zur Trockne eingedampft. Der Rückstand wird in Acetonitril suspendiert und der Feststoff abgesaugt. Die Mutterlauge wird zur Trockne eingedampft, das Produkt mit Dichlormethan extrahiert und mit konz. NaOH gewaschen. Das Produkt wird in Ethanol suspendiert und als Fumarat mit Fumarsäure gefällt. 13 g Produkt werden erhalten. Smp = 179°C

### 11.2 6-Chlor-4-piperazin-1-yl-pyridazin-3-ol (V-2)

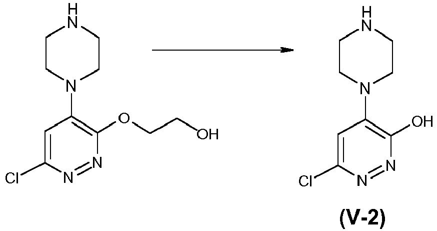

15 g (6-Chlor-4-piperazin-1-yl-pyridazin-3-yloxy)-ethanol Fumarat werden in 90 ml Bromwasserstoff (48%) suspendiert. Das Reaktionsgemisch wird 1 Stunde unter Rückfluß gerührt und zur Trockne eingedampft. 19 g Produkt werden als Hydrobromid erhalten. Smp = 35°C.

### 11.3 6-Chlor-4-{4-[4-(3-fluorphenylamino)-5-oxo-6,7-dihydro-5H-5λ⁴-thieno[3,2-d]pyrimidin-2-yl]-piperazin-1-yl}-pyridazin-3-ol (Beispiel 16)

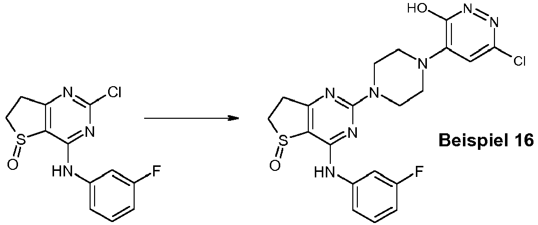

Ausgehend von **(IV-1)** (siehe 1.2) und **(V-2)** (siehe 11.2) wird **Beispiel 16** analog zu Beispiel 15 (siehe 10.5) hergestellt und aufgereinigt. Analytische HPLC-MS (Methode C): RT = 1,86 min.

### 12. SYNTHESE VON (2-{4-[6-(2-ETHOXYETHOXY)-PYRIDAZIN-3-YL]-PIPERAZIN-1-YL}-5-OXO-6,7-DIHYDRO-5H-5λ⁴-THIENO[3,2-d]PYRIMIDIN-4-YL)-(3-FLUORPHENYL)-AMIN TRIFLUORACETAT (BEISPIEL 17)

### 12.1 3-Ethoxyethoxy-6-piperazin-1-yl-pyridazin (V-3)

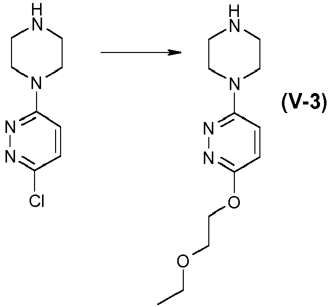

18 g 3-Chlor-6-piperazin-1-yl-pyridazin und 30 g Kaliumhydroxid in 30 ml Wasser werden in 180 ml Ethylglycol suspendiert und 4 Stunden unter Rückfluß gerührt. Das Reaktionsgemisch wird zur Trockne eingedampft. Das Produkt wird mit Diethylether extrahiert, einer konzentrierten Kaliumcarbonat Lösung gewaschen und destilliert (Kp = 190°C). 18 g **(V-3)** werden erhalten.

### 12.2 (2-{4-[6-(2-Ethoxyethoxy)-pyridazin-3-yl]-piperazin-1-yl}-5-oxo-6,7-dihydro-5H-5λ⁴-thieno[3,2-d]pyrimidin-4-yl)-(3-fluorphenyl)-amin (Beispiel 17)

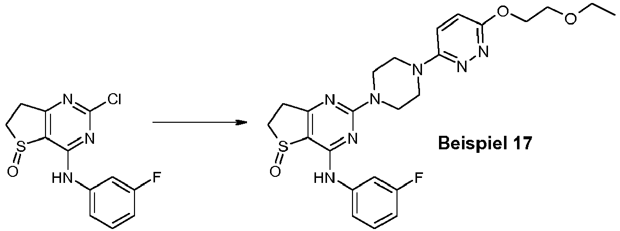

Ausgehend von **(IV-1)** (siehe 1.2) und **(V-3)** (siehe 12.1) wird **Beispiel 17** als Trifluoracetat analog zu Beispiel 15 (siehe 10.5) hergestellt und aufgereinigt. Analytische HPLC-MS (Methode C): RT = 1,66 min.

### 13. SYNTHESE VON (3-FLUORPHENYL)-[5-OXO-2-(4-PYRIDAZIN-4-YL-PIPERAZIN-1-YL)-6,7-DIHYDRO-5H-5λ⁴-THIENO[3,2-d]PYRIMIDIN-4-YL]-AMIN TRIFLUORACETAT (BEISPIEL 18)

### 13.1 4-Piperazin-1-yl-pyridazin (V-4)

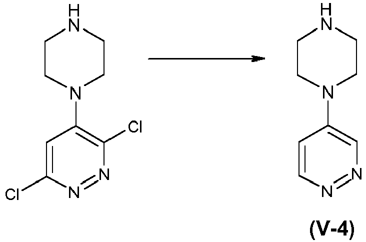

9,3 g 3,6-Dichlor-4-piperazin-1-yl-pyridazin (siehe 10.2) und 6,5 g Natriumacetat werden in 100 ml Methanol suspendiert und mit 1 g Pd/C 10% und Raumtemperatur hydriert. Der Katalysator wird abgesaugt und das Filtrat zur Trockne eingedampft. Das Produkt wird mit Chloroform extrahiert, mit Natronlauge gewaschen und als Hydrochlorid mit einer etherischen HCl Lösung gefällt. 8,6 g **(V-4)** werden erhalten. Smp > 300°C.

### 13.2 (3-Fluorphenyl)-[5-oxo-2-(4-pyridazin-4-yl-piperazin-1-yl)- 6,7-dihydro-5H-5λ⁴-thieno[3,2-d]pyrimidin-4-yl]-amin (Beispiel 18)

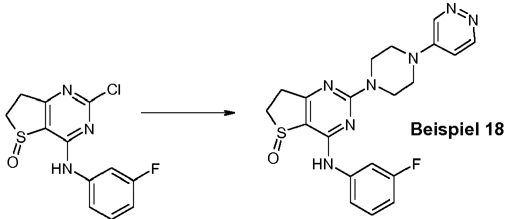

Ausgehend von **(IV-1)** (siehe 1.2) und **(V-4)** (siehe 13.1) wird **Beispiel 18** als Trifluoracetat analog zu Beispiel 15 (siehe 10.5) hergestellt und aufgereinigt. Analytische HPLC-MS (Methode C): RT = 1,54 min.

### 14. SYNTHESE VON (R)-2-{2-[4-(4-METHOXY-1-METHYL-1H-BENZIMIDAZOL-2-YL)-PIPERAZIN-1-YL]-5-OXO-6,7-DIHYDRO-5H-5λ⁴-THIENO[3,2-d]PYRIMIDIN-4-YLAMINO}-3-METHYLBUTAN-1-OL TRIFLUORACETAT (BEISPIEL 23)

### 14.1 (3-Methoxy-2-nitrophenyl)-carbamidsäure tert-butylester

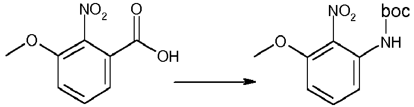

10 g 3-Methoxy-2-nitrobenzoesäure und 7 ml Triethylamin werden in 100 ml *tert*-Butanol vorgelegt und 11 ml Diphenylphosphorylazid zugetropft. Das Reaktionsgemisch wird dann 6 Stunden unter Rückfluß gerührt und zur Trockne eingedampft. Das Produkt wird mit Essigester extrahiert, mit einer10% igen Zitronensäure, einer gesättigten Natriumhydrogencarbonat und einer gesättigten Natriumchlorid Lösung gewaschen. 12,4 g Produkt werden als Feststoff werden erhalten. Smp = 90°C.

### 14.2 (3-Methoxy-2-nitrophenyl)-methyl-carbamidsäure tert-butylester

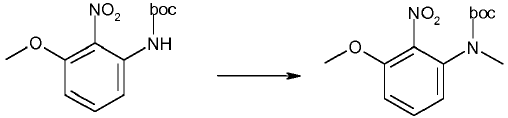

12,2 g (3-Methoxy-2-nitrophenyl)-methyl-carbamidsäure *tert*-butylester werden in 80 ml Dimethylformamid vorgelegt und bei 0°C gekühlt. 2,4 g Natriumhydrid (50% ig in Mineralöl) werden langsam zugegeben. Das Reaktionsgemisch wird 30 Minuten bei 0°C gerührt. Dann werden 3,1 ml Methyliodid zugetropft. Das Reaktionsgemisch wird 2 Stunden bei Raumtemperatur gerührt und mit Wasser versetzt. Das Produkt wird mit Essigester extrahiert. 12.5 g Produkt werden als Öl erhalten.

### 14.3 (3-Methoxy-2-nitrophenyl)-methylamin

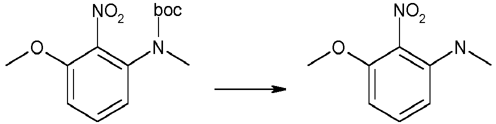

12,5 g (3-Methoxy-2-nitrophenyl)-methyl-carbamidsäure *tert*-butylester und 78 ml Salzsäure (4 M) werden in 300 ml Essigester suspendiert und 5 Stunden bei 60°C erhitzt. Das Reaktionsgemisch wird zur Trockne eingedampft, der Rückstand mit einer gesättigten Natriumhydrogencarbonat Lösung versetzt und das Produkt mit Essigester extrahiert. 7,5 g Produkt werden als Feststoff erhalten. Smp = 58-59°C.

### 14.4 3-Methoxy-N¹-methylbenzol-1,2-diamin

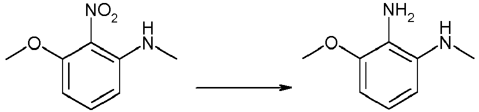

7,4 g (3-Methoxy-2-nitrophenyl)-methylamin werden in 150 ml Essigester suspendiert und mit 1 g Pd/C 10% bei einem Druck von 50 psi und Raumtemperatur hydriert. Nach 4,5 Stunden wird der Katalysator abgesaugt und das Filtrat zur Trockne eingdampft. 5,9 g des Produktes werden als Öl erhalten.

### 14.5 4-Methoxy-1-methyl-1,3-dihydrobenzimidazol-2-on

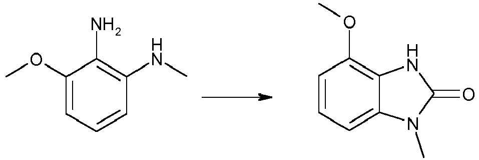

5,9 g 3-Methoxy-N1-methylbenzol-1,2-diamin werden in 70 ml Tetrahydrofuran suspendiert und 6,3 g N,N'-Carbonyldiimidazol zugegeben. Das Reaktionsgemisch wird 5 Stunden bei Raumtemperatur gerührt, mit Wasser versetzt und das Produkt mit Essigester extrahiert. 3,9 g Produkt als Feststoff werden erhalten.

### 14.6 2-Chlor-4-methoxy-1-methyl-1H-benzimidazol

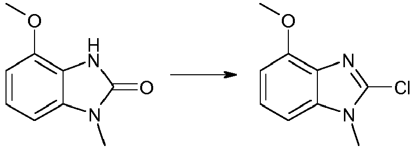

3,7 g 4-Methoxy-1-methyl-1,3-dihydrobenzimidazol-2-on werden in 15 ml Phosphoroxychlorid suspendiert. Das Reaktionsgemisch wird 3 Stunden unter Rückfluß gerührt, langsam mit Eiswasser versetzt und mit konz. Ammoniak alkalisch gestellt. Das ausgefallene Produkt wird abgesaugt. 3,6 g Produkt werden als Feststoff erhalten. Smp = 118 - 119°C.

### 14.7 4-Methoxy-1-methyl-2-piperazin-1-yl-1--benzimidazol (V-5)

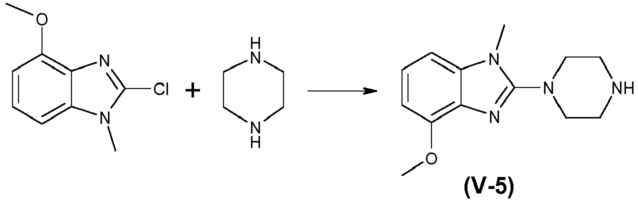

2 g 2-Chlor-4-methoxy-1-methyl-1*H*-benzimidazol und 4,4 g Piperazin werden in 20 ml n-Butanol suspendiert und 5 Stunden unter Rückfluß gerührt. Das Reaktionsgemisch wird zur Trockne eingedampft und das Produkt chromatographisch (Kieselgel, Dichlormethan/ Methanol 10:1) gereinigt. 1,6 g **(V-5)** werden als Feststoff erhalten. Smp = 147°C.

### 14.8 (R)-2-{2-[4-(4-Methoxy-1-methyl-1H-benzimidazol-2-yl)-piperazin-1-yl]-5-oxo-6,7-dihydro-5H-5λ⁴-thieno[3,2-d]pyrimidin-4-ylamino}-3-methylbutan-1-ol (Beispiel 23)

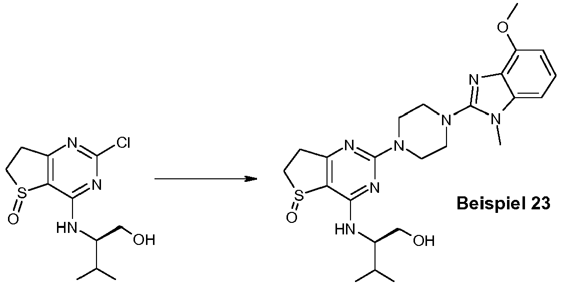

Ausgehend von **(IV-2)** (siehe 2.2) und **(V-5)** (siehe 14.7) wird **Beispiel 23** als trifluoracetat analog zu Beispiel 15 (siehe 10.5) hergestellt und aufgereinigt. Analytische HPLC-MS (Methode C): RT = 1,5 min.

### 15. SYNTHESE VON (R)-2-{2-[4-(7-ETHYL-6,7,8,9-TETRAHYDRO-5H-PYRAZINO[2,3-d]AZEPIN-2-YL)-PIPERAZIN-1-YL]-5-OXO-6,7-DIHYDRO-5H-5λ⁴-THIENO[3,2-d]PYRIMIDIN-4-YLAMINO}-3-METHYLBUTAN-1-OL TRIFLUORACETAT (BEISPIEL 24)

### 15.1 2-Chlor-7-ethyl-6,7,8,9-tetrahydro-5H-pyrazino[2,3-d]azepin

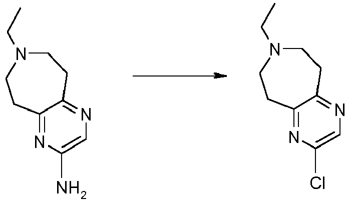

26,5 g 7-Ethyl-6,7,8,9-tetrahydro-5*H*-pyrazino[2,3-*d*]azepin-2-ylamin (US4409220) werden in 130 ml konz. Salzsäure suspendiert, mit 0,1 g Kupfer(I)bromid versetzt und auf -5°C abgekühlt. Eine Suspension von 11 g Natriumnitrit in 14 ml Wasser wird langsam zugetropft. Das Reaktionsgemisch wird 15 Stunden bei Raumtemperatur gerührt und fast zur Trockne eingedampft. Der Rückstand wird langsam auf Eiswasser und Kaliumcarbonat gegeben. Das Produkt wird mit Dichlormethan extrahiert und als Hydrochlorid mit einer etherischen HCl Lösung gefällt. 14,3 g Produkt werden erhalten. Smp = 258 - 262°C

### 15.2 7-Ethyl-2-piperazin-1-yl-6,7,8,9-tetrahydro-5H-pyrazino[2,3-d]azepin (V-6)

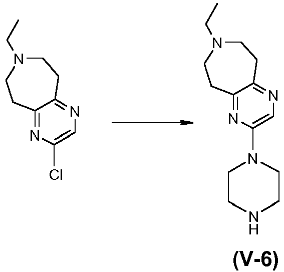

3 g 2-Chlor-7-ethyl-6,7,8,9-tetrahydro-5H-pyrazino[2,3-d]azepin werden mit 23,3 g Piperazin versetzt und 5 Stunden bei 145°C erhitzt. Überschüssiges Piperazin wird abdestilliert und der Rückstand mit Dichlormethan und Methanol behandelt. Ausgefallenes Produkt wird abgesaugt und chromatographisch (Alox, Dioxan/Toluol/Methanol/ NH₄OH 50/20/20/2) gereinigt. 1,95 g Produkt werden erhalten.

### 15.3 (R)-2-{2-[4-(7-Ethyl-6,7,8,9-tetrahydro-5H-pyrazino[2,3-d]azepin-2-yl)-piperazin-1-yl]-5-oxo-6,7-dihydro-5H-5□⁴-thieno[3,2-d]pyrimidin-4-ylamino}-3-methylbutan-1-ol (Beispiel 24)

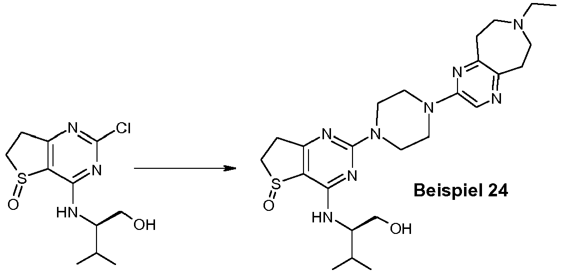

Ausgehend von **(IV-2)** (siehe 2.2) und **(V-6)** (siehe 15.2) wird **Beispiel 24** als Trifluoracetat analog zu Beispiel 15 (siehe 10.5) hergestellt und aufgereinigt. Analytische HPLC-MS (Methode C): RT = 1,38 min.

### 16. SYNTHESE VON (R)-3-METHYL-2-[5-OXO-2-(4-PYRIMIDIN-4-YL-PIPERAZIN-1-YL)-6,7-DIHYDRO-5H-5λ⁴-THIENO[3,2-d]PYRIMIDIN-4-YLAMINO]-BUTAN-1-OL TRIFLUORACETAT (BEISPIEL 28)

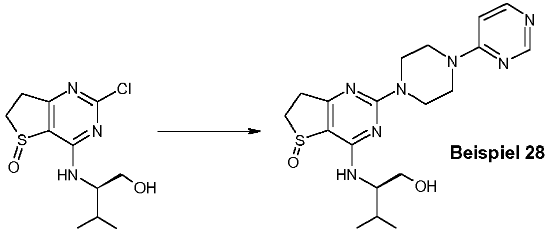

Ausgehend von **(IV-2)** (siehe 2.2) und 4-Piperazin-1-yl-pyrimidin (J. Org. Chem. 1953, 1484) wird **Beispiel 28** als Trifluoracetat analog zu Beispiel 15 (siehe 10.5) hergestellt und aufgereinigt. Analytische HPLC-MS (Methode C): RT = 1,31 min.

### 17. SYNTHESE VON 4-{4-[4-((R)-1-HYDROXYMETHYL-2-METHYLPROPYLAMINO)-5-OXO-6,7-DIHYDRO-5H-5λ⁴-THIENO[3,2-d]PYRIMIDIN-2-YL]-PIPERAZIN-1-YL}-PYRIDIN-2-OL (BEISPIEL 29)

### 17.1 4-(1-Oxypyridin-4-yl)-piperazin-1-BOC:

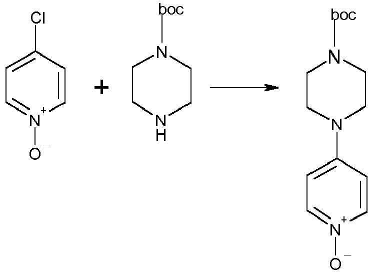

3 g 4-Chlorpyridin-N-oxid und 13,2 g Piperazin-1-BOC werden bei 90°C 4 Stunden erhitzt. Das Produkt wird chromatographisch (Kieselgel, Dichlormethan/Methanol/Ammoniak 90/10/1) gereinigt. 2,9 g Produkt werden als Feststoff erhalten.

### 17.2 4-(2-Hydroxypyridin-4-yl)-piperazin-1-BOC

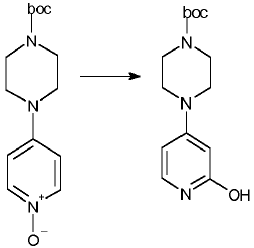

1,75 g 4-(1-Oxypyridin-4-yl)-piperazin-1-BOC werden in 15 ml Essigsäureanhydrid suspendiert und 24 h bei 150°C erhitzt. Das Reaktionsgemisch wird zur Trockne eingedampft und das Produkt chromatographisch (Kieselgel, Essigester/Methanol/Ammoniak 95/5/0,5) gereinigt. 0,51 g Produkt werden als Feststoff erhalten

### 17.3 4-Piperazin-1-yl-pyridin-2-ol (V-7)

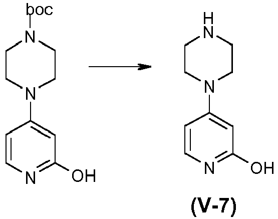

0,51 g 4-(2-Hydroxypyridin-4-yl)-piperazin-1-BOC und 2 ml Trifluoressigsäure werden in 15 ml Dichlormethan suspendiert und 2 Stunden bei Raumtemperatur gerührt. Das Reaktionsgemisch wird zur Trockne eingedampft. 1 g **(V-7)** werden als Öl erhalten. ¹H NMR (400 MHz, DMSO): 7,30 (1 H, d); 5,99 (1 H, dd); 5,34 (1 H, d).

### 17.4 4-{4-[4-((R)-1-Hydroxymethyl-2-methylpropylamino)-5-oxo-6,7-dihydro-5H-5λ⁴-thieno[3,2-d]pyrimidin-2-yl]-piperazin-1-yl}-pyridin-2-ol (Beispiel 29)

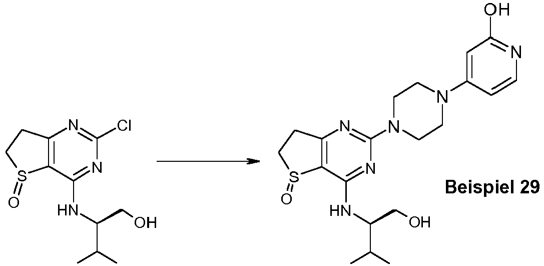

Ausgehend von **(IV-2)** (siehe 2.2) und **(V-7)** (siehe 17.3) wird **Beispiel 29** analog zu Beispiel 15 (siehe 10.5) hergestellt und aufgereinigt. Analytische HPLC-MS (Methode C): RT = 1,37 min.

### 18. SYNTHESE VON (R)-3-METHYL-2-[5-OXO-2-(4-PYRIDIN-4-YL-PIPERAZIN-1-YL)-6,7-DIHYDRO-5H-5λ⁴-THIENO[3,2-d]PYRIMIDIN-4-YLAMINO]-BUTAN-1-OL TRIFLUORACETAT (BEISPIEL 32)

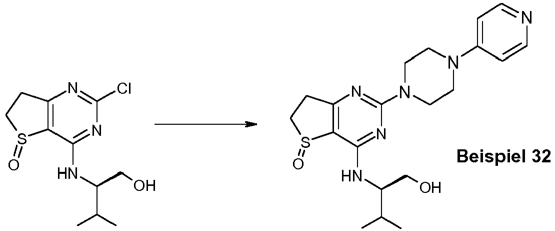

Ausgehend von **(IV-2)** (siehe 2.2) und 1-Pyridin-4-yl-piperazin wird **Beispiel 32** als Trifluoracetat analog zu Beispiel 15 (siehe 10.5) hergestellt und aufgereinigt. Analytische HPLC-MS (Methode C): RT = 1,33 min.

### 19. SYNTHESE VON (R)-2-{2-[4-(3-DIMETHYLAMINOPYRIDAZIN-4-YL)-PIPERAZIN-1-YL]-5-OXO-6,7-DIHYDRO-5H-5λ⁴-THIENO[3,2-d]PYRIMIDIN-4-YLAMINO}-3-METHYLBUTAN-1-OL TRIFLUORACETAT (BEISPIEL 36)

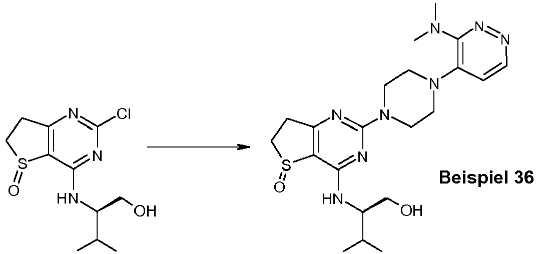

Ausgehend von **(IV-2)** (siehe 2.2) und **(V-1)** (siehe 10.4) wird **Beispiel 36** als Trifluoracetat analog zu Beispiel 15 (siehe 10.5) hergestellt und aufgereinigt. Analytische HPLC-MS (Methode C): RT = 1,37 min.

### 20. SYNTHESE VON 6-CHLOR-4-{4-[4-((R)-1-HYDROXYMETHYL-2-METHYLPROPYLAMINO)-5-OXO-6,7-DIHYDRO-5H-5λ⁴-THIENO[3,2-d]PYRIMIDIN-2-YL]-PIPERAZIN-1-YL}-PYRIDAZIN-3-OL (BEISPIEL 37)

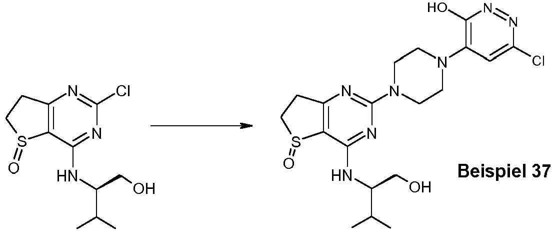

Ausgehend von **(IV-2)** (siehe 2.2) und **(V-2)** (siehe 11.2) wird **Beispiel 37** analog zu Beispiel 15 (siehe 10.5) hergestellt und aufgereinigt. Analytische HPLC-MS (Methode C): RT = 1,55 min.

### 21. SYNTHESE VON (R)-2-(2-{4-[6-(2-ETHOXYETHOXY)-PYRIDAZIN-3-YL]-PIPERAZIN-1-YL}-5-OXO-6,7-DIHYDRO-5H-5λ⁴-THIENO[3,2-d]PYRIMIDIN-4-YLAMINO)-3-METHYLBUTAN-1-OL TRIFLUORACETAT (BEISPIEL 38)

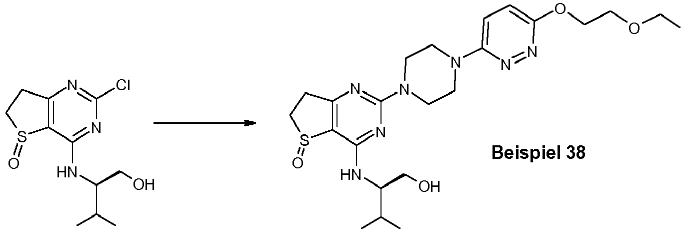

Ausgehend von **(IV-2)** (siehe 2.2) und **(V-3)** (siehe 12.1) wird **Beispiel 38** als Trifluoracetat analog zu Beispiel 15 (siehe 10.5) hergestellt und aufgereinigt. Analytische HPLC-MS (Methode C): RT = 1,45 min.

### 22. SYNTHESE VON (R)-3-METHYL-2-[5-OXO-2-(4-PYRIDAZIN-4-YL-PIPERAZIN-1-YL)-6,7-DIHYDRO-5H-5λ⁴-THIENO[3,2-d]PYRIMIDIN-4-YLAMINO]-BUTAN-1-OL TRIFLUORACETAT (BEISPIEL 39)

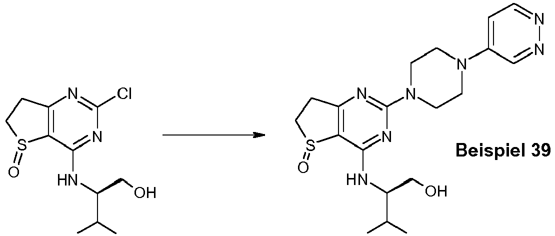

Ausgehend von **(IV-2)** (siehe 2.2) und **(V-4)** (siehe 13.1) wird **Beispiel 39** als Trifluoracetat analog zu Beispiel 15 (siehe 10.5) hergestellt und aufgereinigt. Analytische HPLC-MS (Methode C): RT = 0,56 min.

### 23. SYNTHESE VON {1-[5-OXO-2-(4-PYRIMIDIN-4-YL-PIPERAZIN-1-YL)-6,7-DIHYDRO-5H-5λ⁴-THIENO[3,2-d]PYRIMIDIN-4-YLAMINO]-CYCLOPROPYL}-METHANOL TRIFLUORACETAT (BEISPIEL 55)

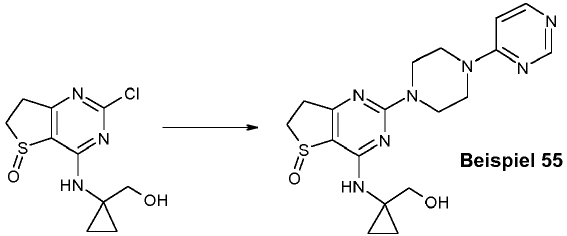

Ausgehend von **(IV-4)** (siehe 8.4) und 4-Piperazin-1-yl-pyrimidin (J. Org. Chem. 1953, 1484) wird **Beispiel 55** als Trifluoracetat analog zu Beispiel 15 (siehe 10.5) hergestellt und aufgereinigt. Analytische HPLC-MS (Methode C): RT = 1,29 min.

### 24 SYNTHESE VON {1-[5-OXO-2-(4-PYRIDIN-4-YL-PIPERAZIN-1-YL)-6,7-DIHYDRO-5H-5λ⁴-THIENO[3,2-d]PYRIMIDIN-4-YLAMINO]-CYCLOPROPYL}-METHANOL TRIFLUORACETAT (BEISPIEL 58)

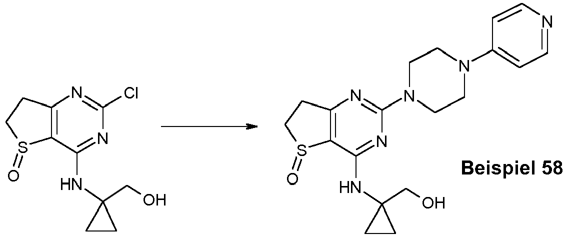

Ausgehend von **(IV-4)** (siehe 8.4) und 1-Pyridin-4-yl-piperazin wird **Beispiel 58** als Trifluoracetat analog zu Beispiel 15 (siehe 10.5) hergestellt und aufgereinigt. Analytische HPLC-MS (Methode C): RT = 1,29 min.

### 25. SYNTHESE VON (S)-1-METHYL-5-[5-OXO-2-(4-PYRIMIDIN-4-YL-PIPERAZIN-1-YL)-6,7-DIHYDRO-5H-5λ⁴-THIENO[3,2-d]PYRIMIDIN-4-YLAMINO]-PIPERIDIN-2-ON TRIFLUORACETAT (BEISPIEL 71)

### 25.1 (S)-5-Dibenzylaminopiperidin-2-on:

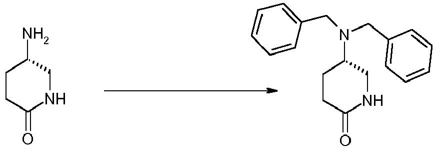

0,600 g 4-(S)-Amino-delta-valerolactam Hydrochlorid, 0,970 ml Benzylbromid und 1,5 g Natriumhydrogencarbonat werden in 30 ml Ethanol suspendiert. Das Reaktionsgemisch wird dann 8 Stunden bei 80°C gerührt und anschießend zur Trockne eingedampft. Der Rückstand wird in Wasser suspensiert und das Produkt mit Dichlormethan extrahiert und chromatographisch (Kieselgel, Dichlormethan/Methanol 100/0 bis 95/5) gereinigt. 0,500 g des Produktes werden als Öl erhalten. Analytische HPLC-MS (Methode A): RT = 1,01 min.

### 25.2 (S)-5-Dibenzylamino-1-methylpiperidin-2-on:

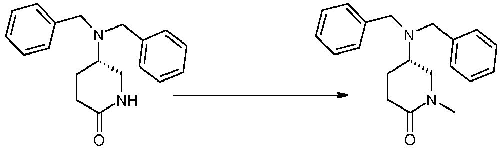

0,500 g (S)-5-Dibenzylaminopiperidin-2-on werden in 15 ml Tetrahydrofuran suspendiert. Unter Eisbadkühlung werden 0,175 g Kalium-*tert*-butylat zugegeben. Das Reaktionsgemisch wird dann 30 Minuten bei Raumtemperatur gerührt. Unter Eisbadkühlung werden 0,095 ml Methyliodid zugegeben. Das Reaktionsgemisch wird dann 48 Stunden bei Raumtemperatur gerührt und anschließend mit einer gesättigten NaCl Lösung versetzt. Das Produkt wird mit Essigester extrahiert. 0,450 g des Produktes werden als Öl erhalten. Analytische HPLC-MS (Methode A): RT = 1,07 min.

### 25.3 (S)-5-Amino-1-methylpiperidin-2-on:

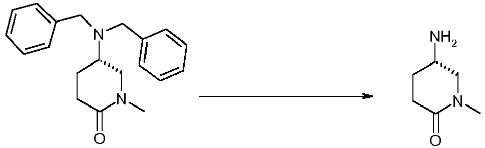

0,450 g (S)-5-Dibenzylamino-1-methylpiperidin-2-on werden in 25 ml Methanol suspendiert und mit 0,150 g Pd/C 10% bei einem Druck von 3 bar und einer Temperatur von 60 °C hydriert. Nach 16 Stunden wird der Katalysator abgesaugt und das Filtrat zur Trockne eingedampft. 0,190 g des Produktes werden als Öl erhalten. ¹H NMR (400 MHz, DMSO): 2,76 (3H, s).

### 25.4 (S)-5-(2-Chlor-6,7-dihydrothieno[3,2-d]pyrimidin-4-ylamino)-1-methylpiperidin-2-on (III-5):

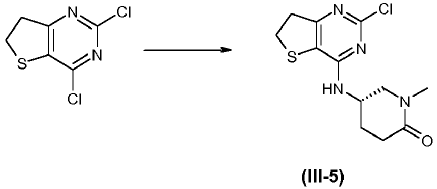

0,27 g **(II)** werden in 3 ml Dioxan vorgelegt, anschliessend werden 0,45 ml Diisopropylethylamin und 0,25 g (S)-5-Amino-1-methylpiperidin-2-on zugegeben. Das Reaktionsgemisch wird bei 130°C, bis keine weitere Umsetzung erfolgt, erhitzt und nach Abkühlen eingedampft. Das Produkt wird mit Dichlormethan extrahiert und chromatographisch (präparative HPLC, Methode B) gereinigt. 0,26 g **(III-5)** werden als Feststoff erhalten. Analytische HPLC-MS (Methode A): RT = 1,06 min.

### 25.5 (S)-5-(2-Chlor-5-oxo-6,7-dihydro-5H-5λ⁴-thieno[3,2-d]pyrimidin-4-ylamino)-1-methylpiperidin-2-on (IV-5):

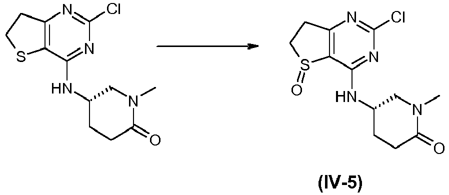

0,04 g S-(-)-1,1'-Bi-2-naphtol werden in 5 ml Chloroform unter Argon vorgelegt, dann 0,02 ml Titan(IV)-isopropylat und 0,025 ml Wasser zugegeben. Das Reaktionsgemisch wird 1 Stunde bei Raumtemperatur gerührt. Anschließend wird eine Suspension von 0,2 g **(III-5)** in 4 ml Dichlormethan zugegeben. Das Reaktionsgemisch wird auf -5°C abgekühlt und nach 20 Minuten werden 0,12 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Das Produkt wird mit Dichlormethan extrahiert und chromatographisch (Kieselgel, Essigester/Methanol 100/0 bis 60/40) gereinigt. 0,09 g **(IV-5)** werden als Feststoff erhalten. Analytische HPLC-MS (Methode A): RT = 0,83 min.

### 25.6 (S)-1-Methyl-5-[5-oxo-2-(4-pyrimidin-4-yl-piperazin-1-yl)- 6,7-dihydro-5H-5λ⁴-thieno[3,2-d]pyrimidin-4-ylamino]-piperidin-2-on (Beispiel 71)

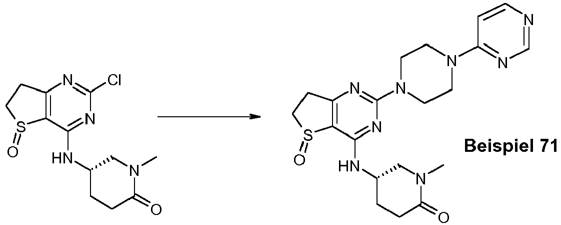

Ausgehend von **(IV-5)** (siehe 25.5) und 4-Piperazin-1-yl-pyrimidin (J. Org. Chem. 1953, 1484) wird **Beispiel 71** als Trifluoracetat analog zu Beispiel 15 (siehe 10.5) hergestellt und aufgereinigt. Analytische HPLC-MS (Methode C): RT = 1,28 min.

### 26. SYNTHESE VON {2-[4-(5-FLUOR-1-METHYL-1H-BENZIMIDAZOL-2-YL)-PIPERAZIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 87)

### 26.1 (4-Fluor-2-nitrophenyl)-methylamin

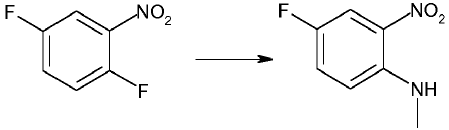

Zu einer 30 ml 40%igen wässrigen Methylamin Lösung werden 7,3 g 1,4-Difluor-2-nitrobenzol unter Eiskühlung langsam zugegeben und das Reaktionsgemisch 1 Stunde bei Raumtemperatur gerührt. Das ausgefallene Produkt wird abgesaugt und aus Wasser und Ethanol umkristallisiert. 6,3 g Produkt werden als Feststoff erhalten. Smp = 74-76°C.

### 26.2 4-Fluor-N¹-methylbenzol-1,2-diamin

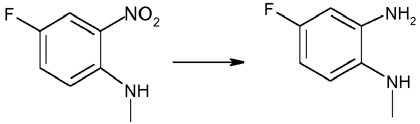

6,2 g (4-Fluor-2-nitrophenyl)-methylamin werden in 200 ml Essigester suspendiert und mit 1 g Raney-Nickel bei einem Druck von 5 bar und Raumtemperatur hydriert. Nach 4,5 Stunden wird der Katalysator abgesaugt und das Filtrat zur Trockne eingedampft. 3,9 g Produkt werden als Öl erhalten.

### 26.3 5-Fluor-1-methyl-1,3-dihydrobenzimidazol-2-on

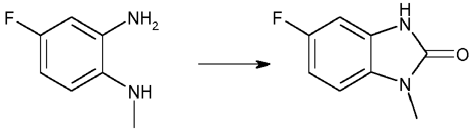

6 g 4-Fluor-N1-methylbenzol-1,2-diamin werden in 200 ml Tetrahydrofuran suspendiert und 7,1 g N,N'-Carbonyldiimidazol zugegeben. Das Reaktionsgemisch wird 48 Stunden bei Raumtemperatur gerührt und das ausgefallene Produkt abgesaugt und aus Dioxan umkristallisiert. 3,9 g Produkt werden als Feststoff erhalten. Smp = 207°C.

### 26.4 2-Chlor-5-fluor-1-methyl-1H-benzimidazol

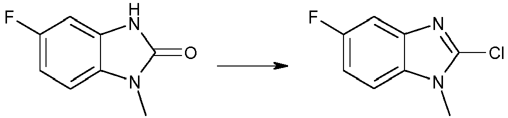

3,9 g 5-Fluor-1-methyl-1,3-dihydro-benzimidazol-2-on werden in 80 ml Phosphoroxychlorid suspendiert und das Reaktionsgemisch 2 Stunden unter Rückfluß gerührt. 50 ml Diethylanilin werden zugegeben. Das Reaktionsgemisch wird weitere 10 Minuten unter Rückfluß gerührt und langsam mit Eiswasser versetzt. Das Produkt wird mit Dichlormethan extrahiert und chromatographisch (Kieselgel, Cyclohexan, Methylenchlorid/Aceton 20/1) gereinigt. 1,4 g Produkt werden als Feststoff erhalten. Smp = 138-141 °C.

### 26.5 5-Fluor-1-methyl-2-piperazin-1-yl-1H-benzimidazol (V-8)

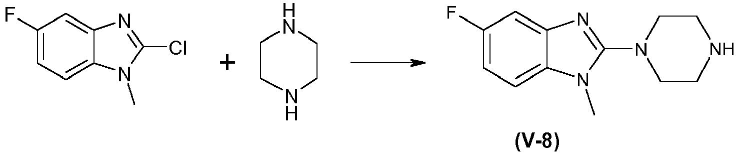

0,7 g 2-Chlor-5-fluor-1-methyl-1*H*-benzimidazol und 1,3 g Piperazin werden in 10 ml n-Butanol suspendiert und 48 Stunden bei Raumtemperatur gerührt. Das Reaktionsgemisch wird zur Trockne eingedampft und das Produkt chromatographisch (Aluminiumoxid, Methylenchlorid/Methanol 10/1) gereinigt. 0,73 g **(V-8)** werden als Feststoff erhalten. ¹H NMR (400 MHz, DMSO): 6,9 (1 H, t); 3,6 (3H, s).

### 26.6 {2-[4-(5-Fluor-1-methyl-1H-benzoimidazol-2-yl)-piperazin-1-yl]-5-oxo-6,7-dihydro-5H-5λ⁴-thieno[3,2-d]pyrimidin-4-yl}-(tetrahydropyran-4-yl)-amin (Beispiel 87)

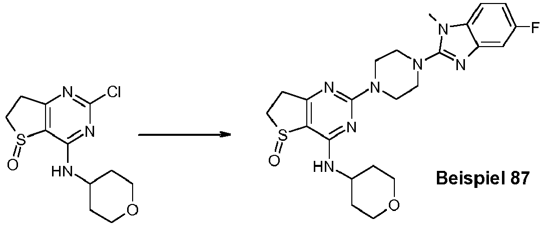

Ausgehend von **(IV-3)** (siehe 4.2) und **(V-8)** (siehe 26.5) wird **Beispiel 87** analog zu Beispiel 15 (siehe 10.5) hergestellt und aufgereinigt. Analytische HPLC-MS (Methode C): RT = 1,48 min.

### 27. SYNTHESE VON [5-OXO-2-(4-PYRIDIN-4-YL-PIPERAZIN-1-YL)-6,7-DIHYDRO-5H-5λ⁴-THIENO[3,2-d]PYRIMIDIN-4-YL]-(TETRAHYDROPYRAN-4-YL)-AMIN TRIFLUORACETAT (BEISPIEL 96)

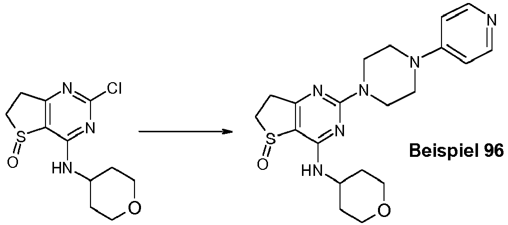

Ausgehend von **(IV-3)** (siehe 4.2) und 1-Pyridin-4-yl-piperazin wird **Beispiel 96** als Trifluoracetat analog zu Beispiel 15 (siehe 10.5) hergestellt und aufgereinigt. Analytische HPLC-MS (Methode C): RT = 1,32 min.

### 28. SYNTHESE VON(3-FLUORPHENYL)-{2-[4-(4-METHOXY-1-METHYL-1H-BENZIMIDAZOL-2-YL)-PIPERAZIN-1-YL]-5-OXO-6,7-DIHYDRO-5H-5λ⁴-THIENO[3,2-d]PYRIMIDIN-4-YL}-AMIN TRIFLUORACETAT (BEISPIEL 108)

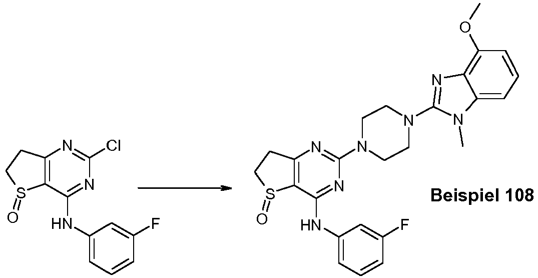

Ausgehend von **(IV-1)** (siehe 1.2) und **(V-5)** (siehe 14.7) wird **Beispiel 108** als Trifluoracetat analog zu Beispiel 15 (siehe 10.5) hergestellt und aufgereinigt. Analytische HPLC-MS (Methode C): RT = 1,73 min.

### 29. SYNTHESE VON {2-[4-(7-ETHYL-6,7,8,9-TETRAHYDRO-5H-PYRAZINO[2,3-d]AZEPIN-2-YL)-PIPERAZIN-1-YL]-5-OXO-6,7-DIHYDRO-5H-5λ⁴-THIENO[3,2-d]PYRIMIDIN-4-YL}-(3-FLUORPHENYL)-AMIN TRIFLUORACETAT (BEISPIEL 109)

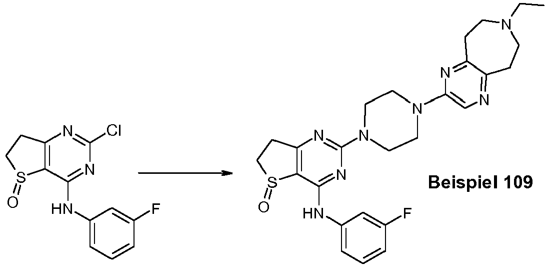

Ausgehend von **(IV-1)** (siehe 1.2) und **(V-6)** (siehe 15.2) wird **Beispiel 109** als Trifluoracetat analog zu Beispiel 15 (siehe 10.5) hergestellt und aufgereinigt. Analytische HPLC-MS (Methode C): RT = 1,6 min.

### 30. SYNTHESE VON (3-FLUORPHENYL)-[5-OXO-2-(4-PYRIMIDIN-4-YL-PIPERAZIN-1-YL)-6,7-DIHYDRO-5H-5λ⁴-THIENO[3,2-d]PYRIMIDIN-4-YL]-AMIN TRIFLUORACETAT (BEISPIEL 113)

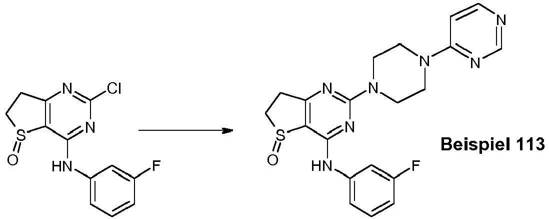

Ausgehend von **(IV-1)** (siehe 1.2) und 4-Piperazin-1-yl-pyrimidin (J. Org. Chem. 1953, 1484) wird **Beispiel 113** als Trifluoracetat analog zu Beispiel 15 (siehe 10.5) hergestellt und aufgereinigt. Analytische HPLC-MS (Methode C): RT = 1,56 min.

### 31. SYNTHESE VON 4-{4-[4-(3-FLUORPHENYLAMINO)-5-OXO-6,7-DIHYDRO-5H-5λ⁴-THIENO[3,2-d]PYRIMIDIN-2-YL]-PIPERAZIN-1-YL}-PYRIDIN-2-OL (BEISPIEL 114)

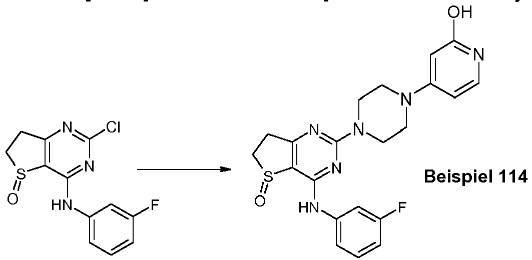

Ausgehend von **(IV-1)** (siehe 1.2) und **(V-7)** (siehe 17.3) wird **Beispiel 114** analog zu Beispiel 15 (siehe 10.5) hergestellt und aufgereinigt. Analytische HPLC-MS (Methode C): RT = 1,61 min.

### 32. SYNTHESE VON (3-FLUORPHENYL)-[5-OXO-2-(4-PYRIDIN-4-YL-PIPERAZIN-1-YL)-6,7-DIHYDRO-5H-5λ⁴-THIENO[3,2-d]PYRIMIDIN-4-YL]-AMIN (BEISPIEL 117)

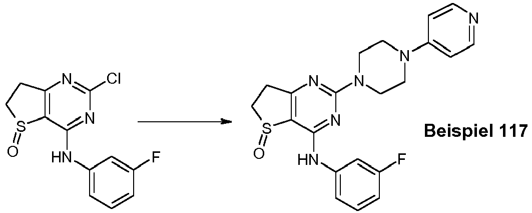

Ausgehend von **(IV-1)** (siehe 1.2) und 1-Pyridin-4-yl-piperazin wird **Beispiel 117** analog zu Beispiel 15 (siehe 10.5) hergestellt und aufgereinigt. Analytische HPLC-MS (Methode C): RT = 1,56 min.

### 33. SYNTHESE VON (3-FLUORPHENYL)-(2-{4-[4-(4-FLUORPHENYL)-THIAZOL-2-YL]-PIPERAZIN-1-YL}-5-OXO-6,7-DIHYDRO-5H-5λ⁴-THIENO[3,2-d]PYRIMIDIN-4-YL)-AMIN (BEISPIEL 142)

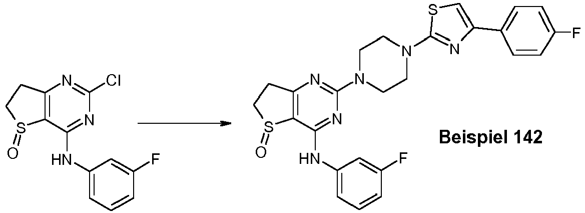

Ausgehend von **(IV-1)** (siehe 1.2) und 1-[4-(4-Fluorphenyl)-thiazol-2-yl]-piperazin wird **Beispiel 142** analog zu Beispiel 15 (siehe 10.5) hergestellt und aufgereinigt. Analytische HPLC-MS (Methode C): RT = 2,42 min.

### 34. SYNTHESE VON [2-(4-BENZO[d]ISOXAZOL-3-YL-PIPERAZIN-1-YL)-5-OXO-6,7-DIHYDRO-5H-5λ⁴-THIENO[3,2-d]PYRIMIDIN-4-YL]-(3-FLUORPHENYL)-AMIN (BEISPIEL 144)

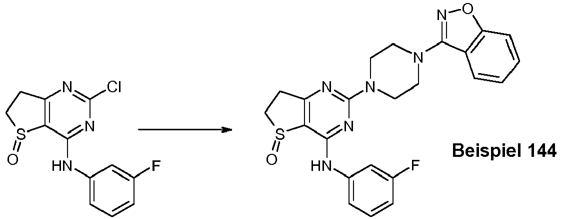

Ausgehend von **(IV-1)** (siehe 1.2) und 3-Piperazin-1-yl-benzo[*d*]isoxazol wird **Beispiel 144** analog zu Beispiel 15 (siehe 10.5) hergestellt und aufgereinigt. Analytische HPLC-MS (Methode C): RT = 2,19 min.

### 35. SYNTHESE VON (R)-2-(2-{4-[4-(4-FLUORPHENYL)-THIAZOL-2-YL]-PIPERAZIN-1-YL}-5-OXO-6,7-DIHYDRO-5H-5λ⁴-THIENO[3,2-d]PYRIMIDIN-4-YLAMINO)-3-METHYLBUTAN-1-OL (BEISPIEL 148)

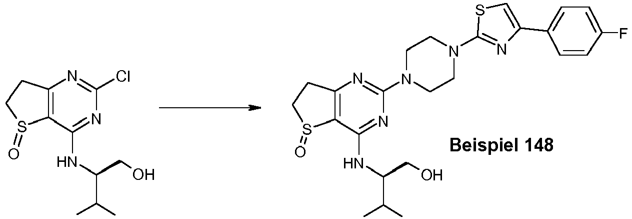

Ausgehend von **(IV-2)** (siehe 2.2) und 1-[4-(4-Fluorphenyl)-thiazol-2-yl]-piperazin wird **Beispiel 148** analog zu Beispiel 15 (siehe 10.5) hergestellt und aufgereinigt. Analytische HPLC-MS (Methode C): RT = 1,91 min.

### 36. SYNTHESE VON (R)-2-[2-(4-BENZO[d]ISOXAZOL-3-YL-PIPERAZIN-1-YL)-5-OXO-6,7-DIHYDRO-5H-5λ⁴-THIENO[3,2-d]PYRIMIDIN-4-YLAMINO]-3-METHYLBUTAN-1-OL (BEISPIEL 150)

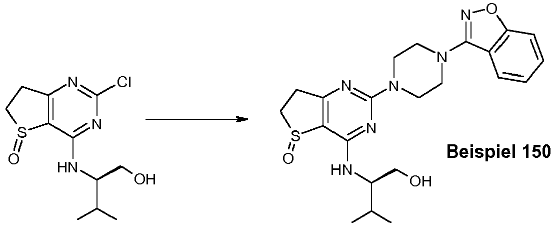

Ausgehend von **(IV-2)** (siehe 2.2) und 3-Piperazin-1-yl-benzo[*d*]isoxazol wird **Beispiel 150** analog zu Beispiel 15 (siehe 10.5) hergestellt und aufgereinigt. Analytische HPLC-MS (Methode C): RT = 1,76 min.

### CHROMATOGRAPHIE METHODEN

Die nach den obigen Synthese-Schema hergestellten Beispielverbindungen wurden durch folgende chromatographische Methoden, die - sofern sie durchgeführt wurden - im einzelnen in der Tabelle A angegeben sind, charakterisiert.

### Analytische HPLC-MS, Methode A

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.50 |
| 0.20 | 95 | 5 | 2.50 |
| 1.50 | 2 | 98 | 2.50 |
| 1.70 | 2 | 98 | 2.50 |
| 1.90 | 95 | 5 | 2.50 |
| 2.20 | 95 | 5 | 2.50 |

Als stationäre Phase dient eine Säule Merck Chromolith™ Flash RP-18e, 4.6 mm x 25 mm (Säulentemperatur: konstant bei 25°C).

### Analytische HPLC-MS, Methode B

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% NH₃
B: Acetonitril mit 0.10% NH₃

| Zeit in min | %A | %B | Flussrate in ml/min |
|---|---|---|---|
| 0.00 | 95 | 5 | 3.00 |
| 0.20 | 95 | 5 | 3.00 |
| 1.50 | 2 | 98 | 3.00 |
| 1.90 | 2 | 98 | 3.00 |
| 2.00 | 2 | 98 | 3.00 |

Als stationäre Phase dient Waters, X-Bridge, C18, 3,5 nm, 4,6 X 20 mm. Raumtemperatur

### Analytische HPLC-MS, Methode C

Waters ZQ2000 Massenspektrometer (positive Ionisation (ESI+)), HP1100 HPLC (DAD, Wellenlängenbereich: 210 to 500 nm), and Gilson 215 Autosampler.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.50 |
| 2.00 | 0 | 100 | 1.50 |
| 2.50 | 0 | 100 | 1.50 |
| 2.60 | 95 | 5 | 1.50 |

Als stationäre Phase dient eine Säule Sunfire C18, 4,6 X 50mm, 3,5 µm, Säulentemperatur 40°C.

### Analytische HPLC, Methode A

Agilent 1100, .Diodenarraydetektion erfolgt im Wellenlängenbereich 210-380 nm.
A: Wasser mit 0.10% TFA
B: Acetonitril mit 0.13% TFA

| Zeit in min | %A | %B | Flussrate in ml/min |
|---|---|---|---|
| 0.00 | 95 | 5 | 1.50 |
| 0.60 | 95 | 5 | 1.50 |
| 3.40 | 2 | 98 | 1.50 |
| 3.90 | 2 | 98 | 1.50 |
| 4.20 | 95 | 5 | 1.50 |
| 4.90 | 95 | 5 | 1.50 |

Als stationäre Phase dient eine Säule Varian Microsorb, RP C18, 3 µm, 100 A, Raumtemperatur.

### Präparative HPLC-MS, Methode A

Waters ZQ2000 Masse Spektrometer (positive Ionisation (ESI+)), HP1100 HPLC (DAD, Wellenlängenbereich: 210 - 500 nm), and Gilson 215 Autosampler.
A: Wasser mit 0.10% TFA
B: Acetonitril

| Zeit in min | %A | %B | Flussrate in ml/min |
|---|---|---|---|
| 0.00 | 90 | 10 | 50 |
| 1.50 | 90 | 10 | 50 |
| 8.00 | 40 | 60 | 50 |
| 10.00 | 40 | 60 | 50 |
| 11.00 | 90 | 10 | 50 |

Als stationäre Phase dient eine Säule Sunfire C18, 30 X 100 mm, 5 µm, Raumtemperatur.

### Präparative HPLC, Methode A

Gilson HPLC mit Gilson UV-VIS-155 Detektor, Sampling Injektor 231 XL.

Als Wellenlänge wird das substanzspezifische UV-Maximum angegeben.
A: Wasser mit 0.1 % Ammoniak 35%ig
B: Acetonitril

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.-50 | 95 | 5 | 180 |

Als stationäre Phase dient eine Säule Pursuit XRS RP 18, 10 µm, 50 X 150 mm, Raumtemperatur.

### Präparative HPLC, Methode B

Gilson HPLC mit Gilson UV-VIS-155 Detektor, Sampling Injektor 231 XL.

Als Wellenlänge wird das substanzspezifische UV-Maximum angegeben.
A: Wasser mit 0.13% TFA
B: Acetonitril mit 0,1% TFA

| Zeit in min | %A | %B | Flussrate in ml/min |
|---|---|---|---|
| 0.00 | 95 | 5 | 165 |
| 1.30 | 95 | 5 | 165 |
| 8.90 | 2 | 98 | 165 |
| 10.00 | 2 | 98 | 165 |
| 10.50 | 95 | 5 | 165 |
| 11.60 | 95 | 5 | 165 |

Als stationäre Phase dient eine Säule Microsorb RP 18, 8 µm, 50 X 65 mm, Raumtemperatur

### BEISPIELE

Die folgenden Beispiele wurden analog den oben gezeigten Synthesevorschriften herstellt (je nach Kennzeichnung in der Tabelle). Diese Verbindungen sind als PDE4-Inhibitoren geeignet und besitzen IC₅₀-Werte kleiner oder gleich 1 µmol. Die Inhibitionen (in %) bei 1 µM der einzelnen Beispiel-Substanzen sind in den nachfolgenden Beispiel-Tabellen eingefügt und wurden wie folgt bestimmt:
Bei der Durchführung des Scintilation Proximity (SPA) Assays (GE Healthcare, Nr. TRKQ7090) werden die unterschiedlichen Affinitäten des cyklischen 3'-5'-Adenosinmonophosphates (cAMP, niedrige Affinität) und des linearen 5'-Adenosinmonophosphates (AMP, hohe Affinität) an Yttrium-Silicat-Scintillatorbeads ausgenutzt. Die cAMP spezifische Phosphodiesterase (PDE) PDE4B spaltet die 3'-Phosphoesterbindung des Tritiummarkierten [H3]-cAMP zum [H3]-5'-AMP. Dieses [H3]-AMP lagert sich aufgrund der höheren Affinität zu den Scintillatorbeads an diese an und verursacht Scintillationsereignisse (Lichtblitze) welche in einem *Wallac Microbeta Scintillation Counter* gemessen werden.

Der Versuch startet mit einer einstündigen Inkubation von [H3]-cAMP mit dem PDE4B Enzym in Assaypuffer bei 30°C, jeweils einmal mit der zu testenden Beispiel-Substanz (in einer Konzentation von 1µM) und einmal ohne die zu testende Beispiel-Substanz.

Nach dieser Inkubation wird die Reaktion durch Zugabe der Beads gestoppt. Die Beads erhalten in den folgenden 45 Minuten Gelegenheit, sich abzusetzen, danach wird im Scintillation Counter gemessen. Ist die Substanz in der Lage, die enzymatische Aktivität der PDE4B zu hemmen, so entsteht während der Inkubationsphase weniger [H3]-AMP und es sind weniger Scintillationsereignisse messbar. Ausgedrückt werden diese Ergebnisse als Prozent Inhibition bei einer Konzentration der Test-Substanz von 1µM.

Bei den Beispielen handelt es sich um Verbindungen der folgenden Formel **A,** 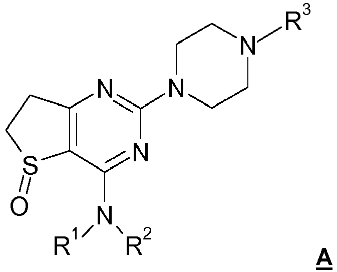 mit den in der folgenden Tabelle A bezeichneten Eigenschaften:

**Tabelle A: Chemische Strukturen und Details zu den Herstellungen der Beispiel-Substanzen 1 - 156**

| # | R¹ | R² | R³ | Herstellung analog zu Beispiel-#* | nicht käuflicher Arylpiperazin-Baustein **(V)** | Literatur zur Herstellung des nicht käuflichen Arylpiperazin-Bausteins **(V)** | Analytische HPLC-MS, RT [min], Methode | % Inhibition PDE4B @ 1 µM |
|---|---|---|---|---|---|---|---|---|
| 1 | H | 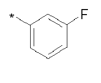 | 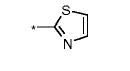 | siehe experim. Teil | | | 1,07 Methode A | 94 |
| 2 | H | 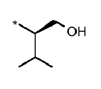 | 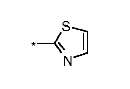 | siehe experim. Teil | | | 0,87 Methode A | 90 |
| 3 | H | 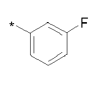 | 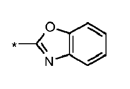 | siehe experim. Teil | | | 1,23 Methode A | 95 |
| 4 | H | 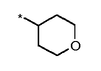 | 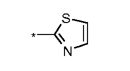 | 5 | | | 0,85 Methode A | 93 |
| 5 | H | 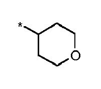 | 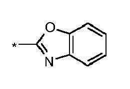 | siehe experim. Teil | | | 1,04 Methode A | 96 |
| 6 | H | 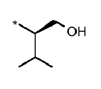 | 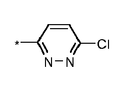 | siehe experim. Teil | | | 0,98 Methode A | 93 |
| 7 | H | 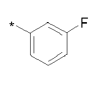 | 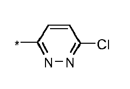 | siehe experim. Teil | | | 1,12 Methode A | 95 |
| 8 | H | 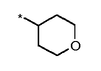 | 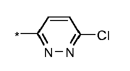 | 5 | | | 0,95 Methode A | 94 |
| 9 | H | 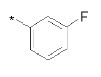 | 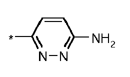 | 1 | | | 1,03 Methode A | 94 |
| 10 | H | 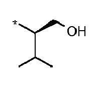 | 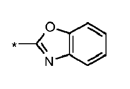 | siehe experim. Teil | | | 1,06 Methode A | 94 |
| 11 | H | 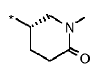 | 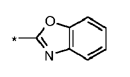 | 71 | | | 1,04 Methode A | 96 |
| 12 | H | 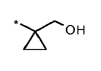 | 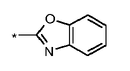 | 13 | | | 1,05 Methode A | 95 |
| 13 | H | 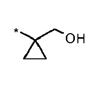 | 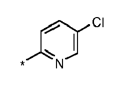 | siehe experim. Teil | | | 1,09 Methode B | 95 |
| 14 | H | 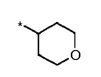 | 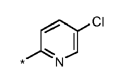 | siehe experim. Teil | | | 1,14 Methode B | 95 |
| 15 | H | 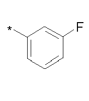 | 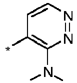 | siehe experim. Teil | | | 1,61 Methode C | 97 |
| 16 | H | 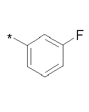 | 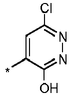 | siehe experim. Teil | | | 1,86 Methode C | 95 |
| 17 | H | 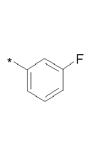 | 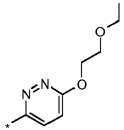 | siehe experim. Teil | | | 1,66 Methode C | 97 |
| 18 | H | 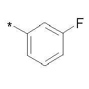 | 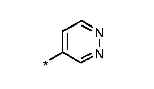 | siehe experim. Teil | | | 1,54 Methode C | 96 |
| 19 | H | 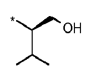 | 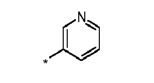 | 23 | | | 1,34 Methode C | 69 |
| 20 | H | 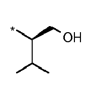 | 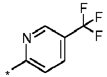 | 23 | | | 1,77 Methode C | 97 |
| 21 | H | 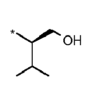 | 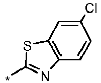 | 23 | | | 1,87 Methode C | 96 |
| 22 | H | 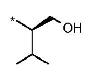 | 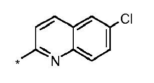 | 23 | | | 1,57 Methode C | 97 |
| 23 | H | 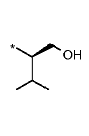 | 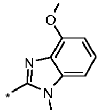 | siehe experim. Teil | | | 1,5 Methode C | 83 |
| 24 | H | 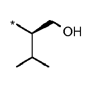 | 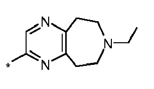 | siehe experim. Teil | | | 1,38 Methode C | 94 |
| 25 | H | 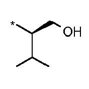 | 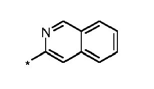 | 23 | 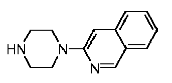 | US4590273 | 1,61 Methode C | 97 |
| 26 | H | 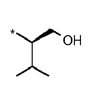 | 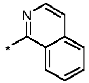 | 23 | | | 1,48 Methode C | 95 |
| 27 | H | 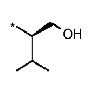 | 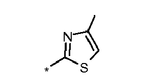 | 23 | | | 1,4 Methode C | 94 |
| 28 | H | 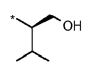 | 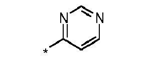 | siehe experim. Teil | | | 1,31 Methode C | 94 |
| 29 | H | 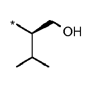 | 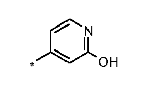 | siehe experim. Teil | | | 1,37 Methode C | 94 |
| 30 | H | 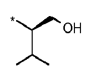 | 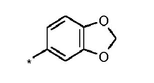 | 23 | | | 1,63 Methode C | 95 |
| 31 | H | 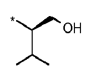 | 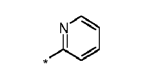 | 23 | | | 1,34 Methode C | 96 |
| 32 | H | 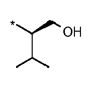 | 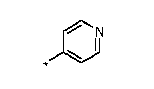 | siehe experim. Teil | | | | 77 |
| 33 | H | 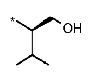 | 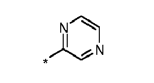 | 23 | | | 1,48 Methode C | 95 |
| 34 | H | 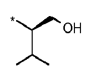 | 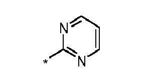 | 23 | | | 1,49 Methode C | 92 |
| 35 | H | 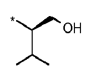 | 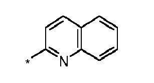 | 23 | | | 1,48 Methode C | 97 |
| 36 | H | 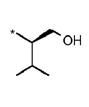 | 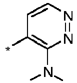 | siehe experim. Teil | | | 1,37 Methode C | 94 |
| 37 | H | 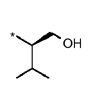 | 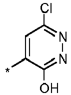 | siehe experim. Teil | | | 1,55 Methode C | 90 |
| 38 | H | 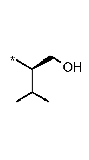 | 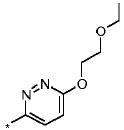 | siehe experim. Teil | | | 1,45 Methode C | 96 |
| 39 | H | 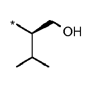 | 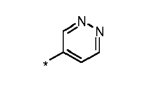 | siehe experim. Teil | | | 0,56 Methode C | 92 |
| 40 | H | 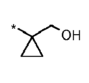 | 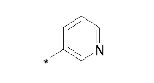 | 55 | | | 1,3 Methode C | 96 |
| 41 | H | 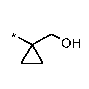 | 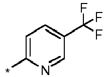 | 55 | | | 1,72 Methode C | 97 |
| 42 | H | 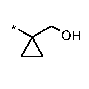 | 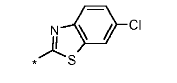 | 55 | | | 1,85 Methode C | 97 |
| 43 | H | 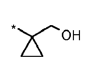 | 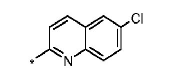 | 55 | | | 1,55 Methode C | 97 |
| 44 | H | 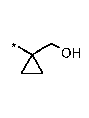 | 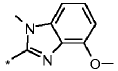 | 55 | 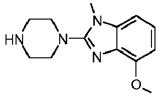 | siehe experim. Teil § 14.7, Bautein **(V-5)** | 1,48 Methode C | 91 |
| 45 | H | 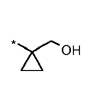 | 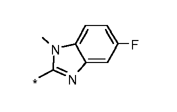 | 55 | 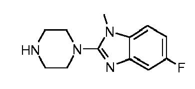 | siehe experim. Teil § 26.5, Bautein **(V-8)** | 1,47 Methode C | 95 |
| 46 | H | 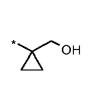 | 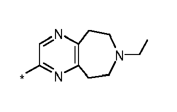 | 55 | 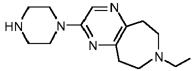 | siehe experim. Teil § 15.2, Bautein **(V-6)** | 1,35 Methode C | 96 |
| 47 | H | 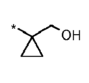 | 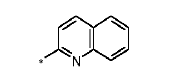 | 55 | | | 1,45 Methode C | 97 |
| 48 | H | 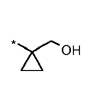 | 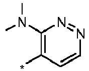 | 55 | 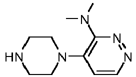 | siehe experim. Teil § 10.4, Bautein **(V-1)** | 1,34 Methode C | 96 |
| 49 | H | 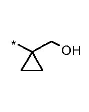 | 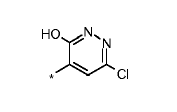 | 55 | 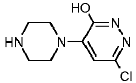 | siehe experim. Teil § 11.2, Bautein **(V-2)** | 1,5 Methode C | 94 |
| 50 | H | 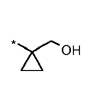 | 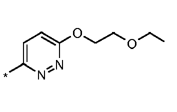 | 55 | 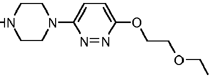 | siehe experim. Teil § 12.1, Bautein **(V-3)** | 1,4 Methode C | 97 |
| 51 | H | 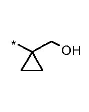 | 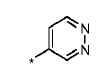 | 55 | 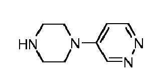 | siehe experim. Teil § 13.1, Bautein **(V-4)** | 0,55 Methode C | 95 |
| 52 | H | 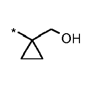 | 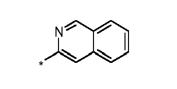 | 55 | 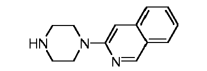 | US4590273 | 1,56 Methode C | 97 |
| 53 | H | 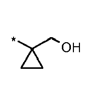 | 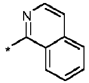 | 55 | | | 1,45 Methode C | 96 |
| 54 | H | 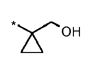 | 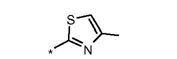 | 55 | | | 1,37 Methode C | 96 |
| 55 | H | 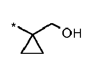 | 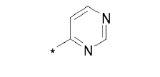 | siehe experim. Teil | | | 1,29 Methode C | 95 |
| 56 | H | 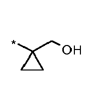 | 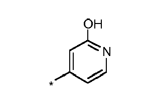 | 55 | 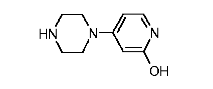 | siehe experim. Teil § 17.3, Bautein **(V-7)** | 1,33 Methode C | 95 |
| 57 | H | 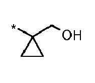 | 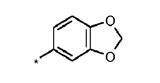 | 55 | | | 1,57 Methode C | 97 |
| 58 | H | 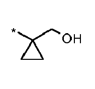 | 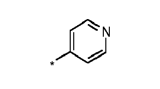 | siehe experim. Teil | | | 1,29 Methode C | 91 |
| 59 | H | 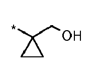 | 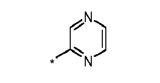 | 55 | | | 1,43 Methode C | 96 |
| 60 | H | 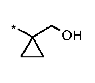 | 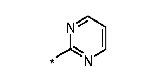 | 55 | | | 1,43 Methode C | 95 |
| 61 | H | 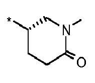 | 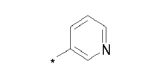 | 71 | | | 1,3 Methode C | 97 |
| 62 | H | 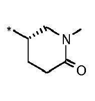 | 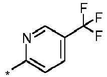 | 71 | | | 1,68 Methode C | 97 |
| 63 | H | 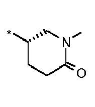 | 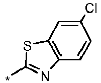 | 71 | | | 1,82 Methode C | 97 |
| 64 | H | 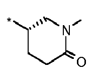 | 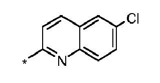 | 71 | | | 1,53 Methode C | 98 |
| 65 | H | 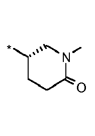 | 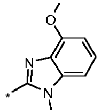 | 71 | 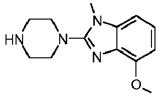 | siehe experim. Teil § 14.7, Bautein **(V-5)** | 1,46 Methode C | 96 |
| 66 | H | 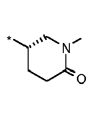 | 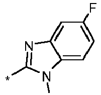 | 71 | 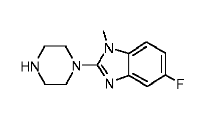 | siehe experim. Teil § 26.5, Bautein **(V-8)** | 1,46 Methode C | 96 |
| 67 | H | 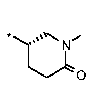 | 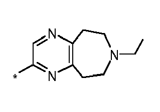 | 71 | 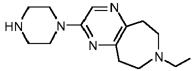 | siehe experim. Teil § 15.2, Bautein **(V-6)** | 1,35 Methode C | 97 |
| 68 | H | 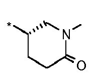 | 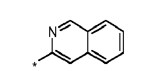 | 71 | 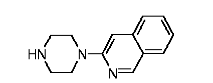 | US4590273 | 1,53 Methode C | 98 |
| 69 | H | 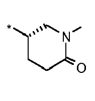 | 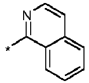 | 71 | | | 1,44 Methode C | 97 |
| 70 | H | 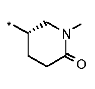 | 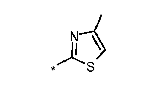 | 71 | | | 1,36 Methode C | 97 |
| 71 | H | 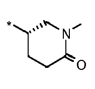 | 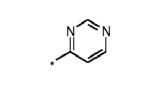 | siehe experim. Teil | 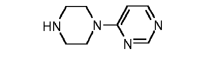 | J. Org. Chem. 1953, 1484 | 1,28 Methode C | 96 |
| 72 | H | 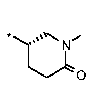 | 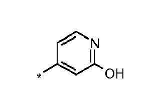 | 71 | 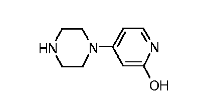 | siehe experim. Teil § 17.3, Bautein **(V-7)** | 1,31 Methode C | 97 |
| 73 | H | 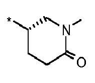 | 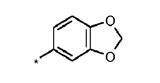 | 71 | | | 1,53 Methode C | 97 |
| 74 | H | 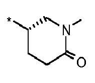 | 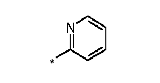 | 71 | | | 1,3 Methode C | 97 |
| 75 | H | 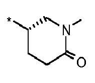 | 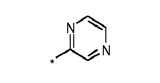 | 71 | | | 1,41 Methode C | 96 |
| 76 | H | 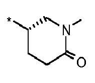 | 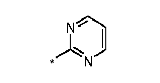 | 71 | | | 1,4 Methode C | 97 |
| 77 | H | 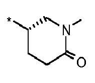 | 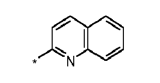 | 71 | | | 1,44 Methode C | 97 |
| 78 | H | 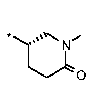 | 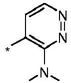 | 71 | 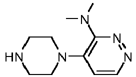 | siehe experim. Teil § 10.4, Bautein **(V-1)** | 1,33 Methode C | 97 |
| 79 | H | 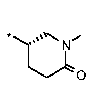 | 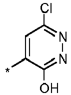 | 71 | 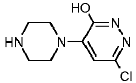 | siehe experim. Teil § 11.2, Bautein **(V-2)** | 1,47 Methode C | 96 |
| 80 | H | 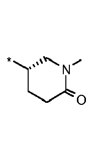 | 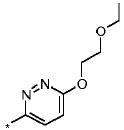 | 71 | 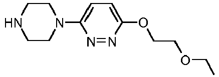 | siehe experim. Teil § 12.1, Bautein **(V-3)** | 1,4 Methode C | 97 |
| 81 | H | 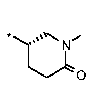 | 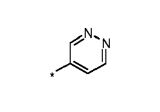 | 71 | 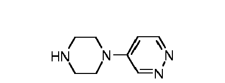 | siehe experim. Teil § 13.1, Bautein **(V-4)** | 1,25 Methode C | 97 |
| 82 | H | 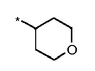 | 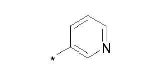 | 87 | | | 1,33 Methode C | 96 |
| 83 | H | 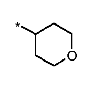 | 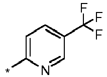 | 87 | | | 1,77 Methode C | 97 |
| 84 | H | 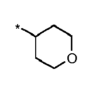 | 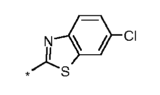 | 87 | | | 1,89 Methode C | 97 |
| 85 | H | 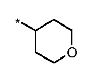 | 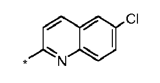 | 87 | | | 1,56 Methode C | 97 |
| 86 | H | 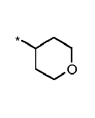 | 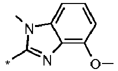 | 87 | 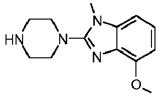 | siehe experim. Teil § 14.7, Bautein **(V-5)** | 1,5 Methode C | 92 |
| 87 | H | 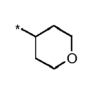 | 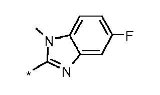 | siehe experim. Teil | | | 1,48 Methode C | 96 |
| 88 | H | 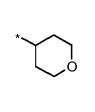 | 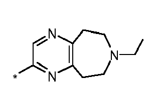 | 87 | 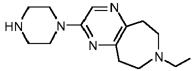 | siehe experim. Teil § 15.2, Bautein **(V-6)** | 1,38 Methode C | 96 |
| 89 | H | 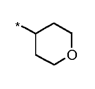 | 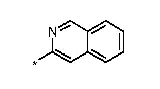 | 87 | 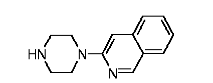 | US4590273 | 1,59 Methode C | 97 |
| 90 | H | 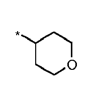 | 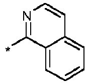 | 87 | | | 1,47 Methode C | 96 |
| 91 | H | 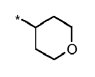 | 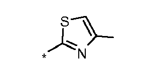 | 87 | | | 1,4 Methode C | 96 |
| 92 | H | 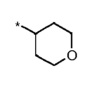 | 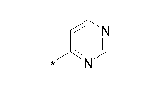 | 87 | 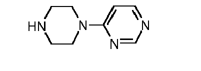 | J. Org. Chem. 1953, 1484 | 1,3 Methode C | 96 |
| 93 | H | 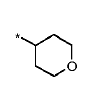 | 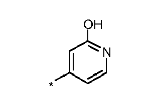 | 87 | 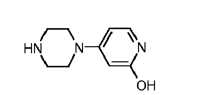 | siehe experim. Teil § 17.3, Bautein **(V-7)** | 1,36 Methode C | 95 |
| 94 | H | 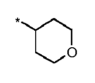 | 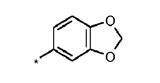 | 87 | | | 1,62 Methode C | 97 |
| 95 | H | 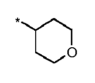 | 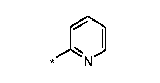 | 87 | | | 1,33 Methode C | 96 |
| 96 | H | 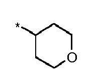 | 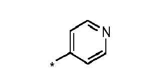 | siehe experim. Teil | | | 1,32 Methode C | 93 |
| 97 | H | 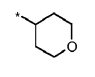 | 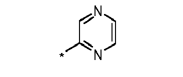 | 87 | | | 1,46 Methode C | 96 |
| 98 | H | 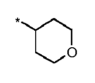 | 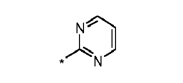 | 87 | | | 1,47 Methode C | 96 |
| 99 | H | 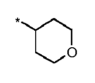 | 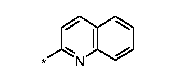 | 87 | | | 1,46 Methode C | 97 |
| 100 | H | 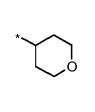 | 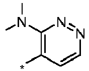 | 87 | 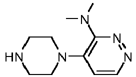 | siehe experim. Teil § 10.4, Bautein **(V-1)** | 1,37 Methode C | 96 |
| 101 | H | 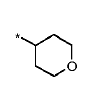 | 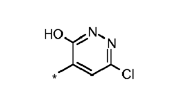 | 87 | 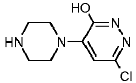 | siehe experim. Teil § 11.2, Bautein **(V-2)** | 1,53 Methode C | 95 |
| 102 | H | 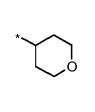 | 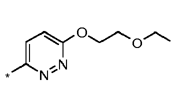 | 87 | 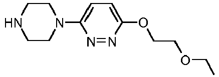 | siehe experim. Teil § 12.1, Bautein **(V-3)** | 1,43 Methode C | 96 |
| 103 | H | 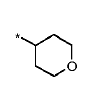 | 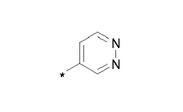 | 87 | 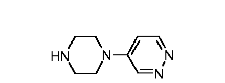 | siehe experim. Teil § 13.1, Bautein **(V-4)** | 1,28 Methode C | 96 |
| 104 | H | 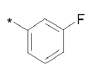 | 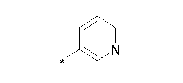 | 108 | | | 1,57 Methode C | 97 |
| 105 | H | 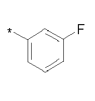 | 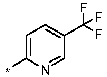 | 108 | | | 2,2 Methode C | 96 |
| 106 | H | 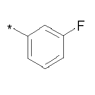 | 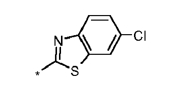 | 108 | | | 2,4 Methode C | 96 |
| 107 | H | 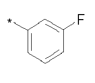 | 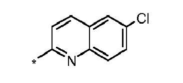 | 108 | | | 1,8 Methode C | 96 |
| 108 | H | 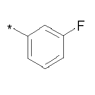 | 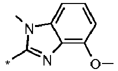 | siehe experim. Teil | | | 1,73 Methode C | 95 |
| 109 | H | 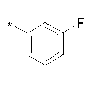 | 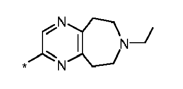 | siehe experim. Teil | | | 1,6 Methode C | 96 |
| 110 | H | 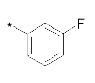 | 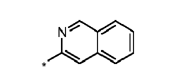 | 108 | 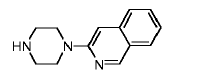 | US4590273 | 1,91 Methode C | 97 |
| 111 | H | 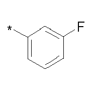 | 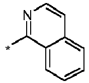 | 108 | | | 1,72 Methode C | 96 |
| 112 | H | 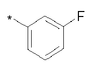 | 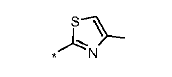 | 108 | | | 1,65 Methode C | 97 |
| 113 | H | 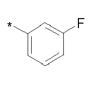 | 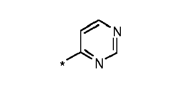 | siehe experim. Teil | | | 1,56 Methode C | 96 |
| 114 | H | 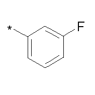 | 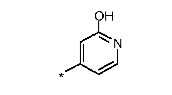 | siehe experim. Teil | | | 1,61 Methode C | 96 |
| 115 | H | 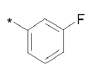 | 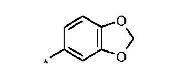 | 108 | | | 1,89 Methode C | 96 |
| 116 | H | 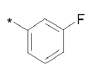 | 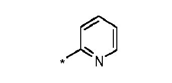 | 108 | | | 1,58 Methode C | 97 |
| 117 | H | 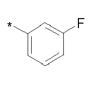 | 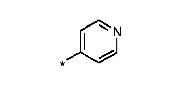 | siehe experim. Teil | | | 1,56 Methode C | 96 |
| 118 | H | 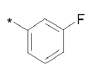 | 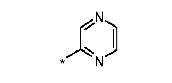 | 108 | | | 1,81 Methode C | 97 |
| 119 | H | 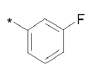 | 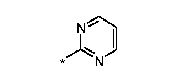 | 108 | | | 1,8 Methode C | 96 |
| 120 | H | 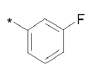 | 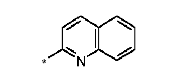 | 108 | | | 1,71 Methode C | 97 |
| 121 | H | 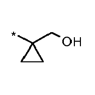 | 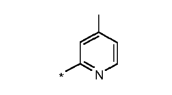 | 55 | | | 1,38 Methode C | 95 |
| 122 | H | 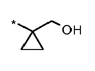 | 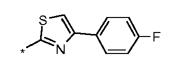 | 55 | | | 1,89 Methode C | 97 |
| 123 | H | 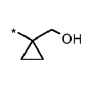 | 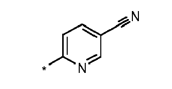 | 55 | | | 1,6 Methode C | 95 |
| 124 | H | 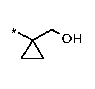 | 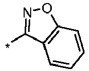 | 55 | | | 1,72 Methode C | 96 |
| 125 | H | 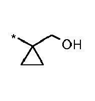 | 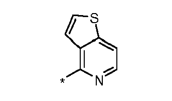 | 55 | | | 1,43 Methode C | 94 |
| 126 | H | 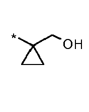 | 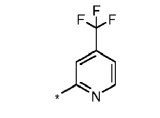 | 55 | | | 1,67 Methode C | 95 |
| 127 | H | 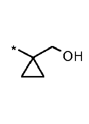 | 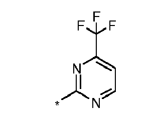 | 55 | | | 1,8 Methode C | 94 |
| 128 | H | 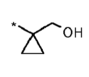 | 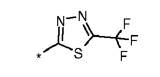 | 55 | | | 1,73 Methode C | 94 |
| 129 | H | 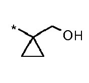 | 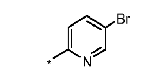 | 55 | | | 1,75 Methode C | 96 |
| 130 | H | 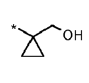 | 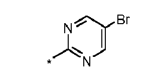 | 55 | | | 1,73 Methode C | 95 |
| 131 | H | 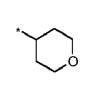 | 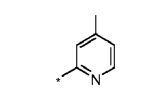 | 87 | | | 1,4 Methode C | 95 |
| 132 | H | 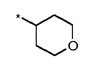 | 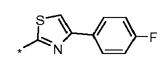 | 87 | | | 1,8 Methode C | 96 |
| 133 | H | 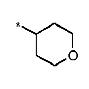 | 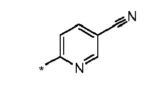 | 87 | | | 1,64 Methode C | 96 |
| 134 | H | 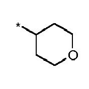 | 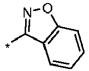 | 87 | | | 1,75 Methode C | 96 |
| 135 | H | 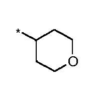 | 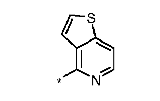 | 87 | | | 1,46 Methode C | 94 |
| 136 | H | 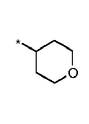 | 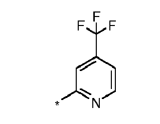 | 87 | | | 1,72 Methode C | 96 |
| 137 | H | 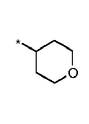 | 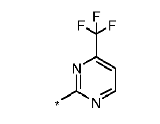 | 87 | | | 1,84 Methode C | 94 |
| 138 | H | 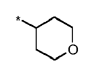 | 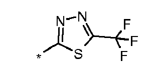 | 87 | | | 1,77 Methode C | 95 |
| 139 | H | 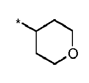 | 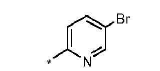 | 87 | | | 1,65 Methode C | 97 |
| 140 | H | 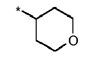 | 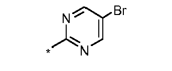 | 87 | | | 1,77 Methode C | 97 |
| 141 | H | 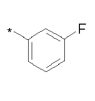 | 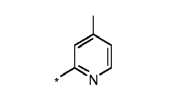 | 108 | | | 1,65 Methode C | 96 |
| 142 | H | 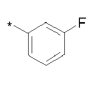 | 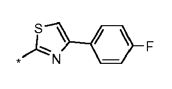 | siehe experim. Teil | | | 1,42 Methode C | 95 |
| 143 | H | 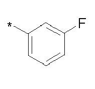 | 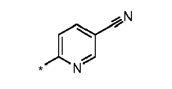 | 108 | | | 2,04 Methode C | 96 |
| 144 | H | 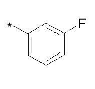 | 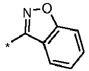 | siehe experim. Teil | | | 2,19 Methode C | 96 |
| 145 | H | 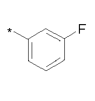 | 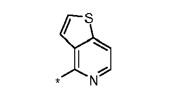 | 108 | | | 1,7 Methode C | 95 |
| 146 | H | 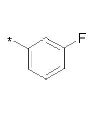 | 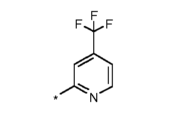 | 108 | | | 2,12 Methode C | 95 |
| 147 | H | 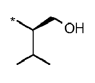 | 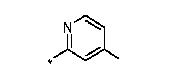 | 23 | | | 1,41 Methode C | 94 |
| 148 | H | 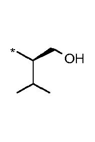 | 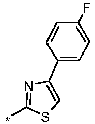 | siehe experim. Teil | | | 1,91 Methode C | 95 |
| 149 | H | 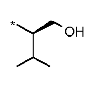 | 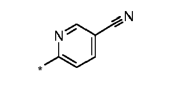 | 23 | | | 1,63 Methode C | 95 |
| 150 | H | 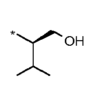 | 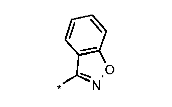 | siehe experim. Teil | | | 1,76 Methode C | 95 |
| 151 | H | 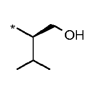 | 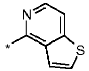 | 23 | | | 1,45 Methode C | 92 |
| 152 | H | 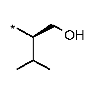 | 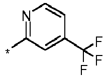 | 23 | | | 1,73 Methode C | 94 |
| 153 | H | 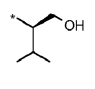 | 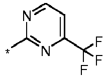 | 23 | | | 1,84 Methode C | 91 |
| 154 | H | 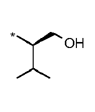 | 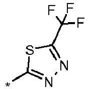 | 23 | | | 1,76 Methode C | 94 |
| 155 | H | 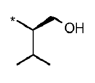 | 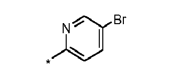 | 23 | | | 1,74 Methode C | 95 |
| 156 | H | 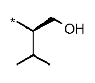 | 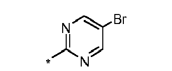 | 23 | | | 1,77 Methode C | 95 |

| | | | | | | | | |
|---|---|---|---|---|---|---|---|---|
| * Das Beispiel kann analog hergestellt und aufgereinigt werden. | | | | | | | | |

### INDIKATIONSGEBIETE

Wie gefunden wurde, zeichnen sich die Verbindungen der Formel **A** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **1** aufgrund ihrer pharmazeutischen Wirksamkeit als PDE4-Inhibitor bevorzugt zur Anwendung gelangen können. Beispielhaft genannt seinen Atemwegs- oder gastrointestinalen Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers' Krankheit, hyperreaktive Atemwege, infektiöse Bronchitis oder Pneumonitis, pediatrisches Asthma, Bronchiectasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alpha1-Antitrypsin-Mangel.

Ebenfalls bevorzugt genannt sei die Behandlung von entzündlichen Erkrankungen des Gastrointestinaltraktes. Beispielhaft hierfür seinen genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seinen genannt alle Formen von akuten und chronischen Leukämien wie, akute lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Besonders bevorzugt betrifft die vorliegende Erfindung die Verwendung von Verbindungen der Formel **A** zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, Morbus Crohn, Colitis ulcerosa, insbesondere COPD, chronische Bronchitis und Asthma.

Am meisten bevorzugt ist die Verwendung der Verbindungen der Formel **A** zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der Verbindungen der Formel **A** zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein herausragender Aspekt der vorliegenden Erfindung ist das reduzierte Profil an Nebenwirkungen. Darunter wird im Rahmen der Erfindung verstanden, eine Dosis einer pharmazeutischen Zusammensetzung verabreichen zu können, ohne beim Patienten Erbrechen, bevorzugt Übelkeit, besonders bevorzugt Unwohlsein auszulösen. Höchst bevorzugt ist die Verabreichung einer therapeutisch wirksamen Substanzmengen, ohne Emesis oder Nausea auszulösen, in jedem Stadium des Krankheitsverlaufs.

### KOMBINATIONEN

Die Verbindungen der Formel **A** können allein oder in Kombination mit anderen erfindungsgemäßen Wirkstoffen der Formel **A** zur Anwendung gelangen. Gegebenenfalls können die Verbindungen der Formel **A** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, weiteren PDE4-Inhibitoren, LTD4-Antagonisten, EGFR-Hemmern, MRP4-Inhibitoren, Dopamin-Agonisten, H1-Antihistaminika, PAF-Antagonisten und PI3-Kinase Inhibitoren oder zwei- oder dreifach Kombinationen davon, wie beispielsweise Kombinationen von Verbindungen der Formel **A** mit ein oder zwei Verbindungen aus der Gruppe bestehend aus
- Betamimetika, Corticosteroiden, PDE4-Inhibitoren, EGFR-Hemmer und LTD4-Antagonisten,
- Anticholinergika, Betamimetika, Corticosteroiden, PDE4-Inhibitoren, EGFR-Hemmern und LTD4-Antagonisten,
- PDE4-Inhibitoren, Corticosteroiden, EGFR-Hemmern und LTD4-Antagonisten
- EGFR-Hemmern, PDE4-inhibitoren und LTD4-Antagonisten
- EGFR-Hemmern und LTD4-Antagonisten
- CCR3-Inhibitoren, iNOS-Inhibitoren (inducible nitric oxide synthase-Inhibitoren), (6R)-L-erythro-5,6,7,8-tetrahydrobiopterin (im folgenden "BH4" genannt) und dessen Derivate wie in WO 2006/120176 genannt und SYK-Inhibitoren (spleen tyrosine kinase-Inhibitoren)
- Anticholinergika, Betamimetika, Corticosteroiden, PDE4-Inhibitoren und MRP4-Inhibitoren.

Auch die Kombinationen dreier Wirkstoffe je einer der o.g. Verbindungsklassen ist Bestandteil der Erfindung.

Als Betamimetika gelangen hierbei vorzugsweise Verbindungen zur Anwendung, die ausgewählt sind aus der Gruppe bestehend aus Albuterol, Bambuterol, Bitolterol, Broxaterol, Carbuterol, Clenbuterol, Fenoterol, Formoterol, Arformoterol, Zinterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenol, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzyl-sulfonamid, 5-[2-(5,6-Diethyl-indan-2-ylamino)-1-hydroxy-ethyl]-8-hydroxy-1H-quinolin-2-on, 4-Hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-Ethoxy-carbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Bevorzugt sind die Betamimetika ausgewählt aus der Gruppe bestehend aus Bambuterol, Bitolterol, Carbuterol, Clenbuterol, Fenoterol, Formoterol, Hexoprenaline, Ibuterol, Pirbuterol, Procaterol, Reproterol, Salmeterol, Sulphonterol, Terbutaline, Tolubuterol, 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1H-quinolin-2-on , 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-ethoxycarbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Besonders bevorzugte Betamimetika sind ausgewählt aus der Gruppe bestehend aus Fenoterol, Formoterol, Salmeterol, 3-(4-{6-[2-Hydroxy-2-(4-hydroxy-3-hydroxymethyl-phenyl)-ethylamino]-hexyloxy}-butyl)-benzenesulfoneamid, 5-[2-(5,6-Diethyl-indan-2-ylamino)-1-hydroxy-ethyl]-8-hydroxy-1 H-quinolin-2-on, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Von diesen Betamimetika sind erfindungsgemäß besonders bevorzugt Formoterol, Salmeterol, 3-(4-{6-[2-Hydroxy-2-(4-hydroxy-3-hydroxymethyl-phenyl)-ethylamino]-hexyloxy}-butyl)-benzenesulfoneamide, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 5-[2-(5,6-Diethyl-indan-2-ylamino)-1-hydroxy-ethyl]-8-hydroxy-1 H-quinolin-2-on, gegebenenfalls in Form ihrer Racemate, Enantiomere, Diasteromere und gegebenenfalls in Form ihrer pharmakologisch verträglichen Säureadditionssalze, Solvate oder Hydrate.

Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Als Anticholinergika gelangen hierbei vorzugsweise Verbindungen zur Anwendung, die ausgewählt sind aus der Gruppe bestehend aus Tiotropiumsalzen, Oxitropiumsalzen, Flutropiumsalzen, Ipratropiumsalzen, Glycopyrroniumsalzen, Trospiumsalzen 2,2-Diphenylpropionsäuretropenolester-methobromid, 2,2-Diphenylpropionsäurescopinester-methobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäure-scopinester-Methobromid, 9-Hydroxy-fluoren-9-carbonsäuretropenolester -Methobromid, 9-Fluor-fluoren-9-carbonsäuretropenolester -Methobromid, 9-Hydroxy-fluoren-9-carbonsäure-scopinester-Methobromid, 9-Fluor-fluoren-9-carbonsäurescopinester Methobromid, 9-Methyl-fluoren-9-carbonsäuretropenolester Methobromid, 9-Methyl-fluoren-9-carbonsäure-scopinester Methobromid, Benzilsäurecyclopropyltropinester-Methobromid, 2,2-Diphenylpropionsäurecyclopropyltropinester -Methobromid, 9-Hydroxy-xanthen-9-carbonsäure-cyclopropyltropinesterMethobromid, 9-Methyl-fluoren-9-carbonsäurecyclopropyltropinester-Methobromid, 9-Methyl-xanthen-9-carbonsäurecyclopropyltropinester -Methobromid, 9-Hydroxy-fluoren-9-carbonsäurecyclopropyltropinester -Methobromid, 4,4'-Difluorbenzilsäuremethylestercyclopropyltropinester -Methobromid, 9-Hydroxy-xanthen-9-carbonsäure-tropenolester-Methobromid, 9-Hydroxy-xanthen-9-carbonsäurescopinester Methobromid, 9-Methyl-xanthen-9-carbonsäuretropenolester -Methobromid, 9-Methyl-xanthen-9-carbonsäurescopinester -Methobromid, 9-Ethyl-xanthen-9-carbonsäuretropenolester Methobromid, 9-Difluormethyl-xanthen-9-carbonsäuretropenolester -Methobromid. 9-Hydroxymethyl-xanthen-9-carbonsäurescopinester -Methobromid, gegebenenfalls in Form ihrer Solvate oder Hydrate.

In den vorstehend genannten Salzen stellen die Kationen Tiotropium, Oxitropium, Flutropium, Ipratropium, Glycopyrronium und Trospium die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodid und Methansulfonat besonders bevorzugt.

Von besonderer Bedeutung ist das Tiotropiumbromid. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Deflazacort,RPR-106541, NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Flunisolid, Beclomethason, Triamcinolon, Budesonid, Fluticason, Mometason, Ciclesonid, Rofleponid, Dexamethason,NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Budesonid, Fluticason, Mometason, Ciclesonid und 6,9-Difluoro-17-[(2-furanylcarbonyl)oxy]-11-hydroxy-16-methyl-3-oxo-androsta-1,4-dien-17-carbothionsäure (S)-fluoromethylester, gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Als weitere PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370,N-(3,5-Dichloro-1-oxo-pyridin-4-yl)-4-difluoromethoxy-3-cyclopropylmethoxybenzamid, (-)p-[(4aR*,10bS*)-9-Ethoxy-1,2,3,4,4a,10b-hexahydro-8-methoxy-2-methyl-benzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Enprofyllin, Roflumilast, Ariflo (Cilomilast), Arofyllin, Atizoram,AWD-12-281 (GW-842470), T-440, T-2585, PD-168787, V-11294A, CI-1018, CDC-801, D-22888, YM-58997, Z-15370, N-(3,5-Dichloro-1-oxo-pyridin-4-yl)-4-difluoromethoxy-3-cyclopropylmethoxybenzamid, Cis[4-Cyano-4-(3-cyclopentyloxy-4-methoxyphenyl)cyclohexan-1-carbonsäure], 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Roflumilast, Ariflo (Cilomilast), Arofyllin,AWD-12-281 (GW-842470), 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], Atizoram, Z-15370, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin gegebenenfalls in Form ihrer Racemate, Enantiomere oder Diastereomere und gegebenenfalls in Form ihrer pharmakologisch verträglichen Säureadditionssalze, Solvate und/oder Hydrate.

Unter Säureadditionssalzen mit pharmakologisch verträglichen Säuren zu deren Bildung die o.g. PDE4-inhibitoren gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321, 1-(((R)-(3-(2-(6,7-Difluoro-2-quinolinyl)ethenyl)phenyl)-3-(2-(2- hydroxy-2-propyl)phenyl)thio)-methylcyclopropan-essigsäure, 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropan-essigsäure und [2-[[2-(4-tert-Butyl-2-thiazolyl)-5-benzofuranyl]oxymethyl]phenyl]essigsäure gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Bevorzugt ist der LTD4-Antagonist ausgewählt aus der Gruppe bestehend aus Montelukast, Pranlukast, Zafirlukast, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Besonders bevorzugt ist der LTD4-Antagonist ausgewählt aus der Gruppe bestehend aus Montelukast, Pranlukast, Zafirlukast, MCC-847 (ZD-3523), MN-001 und MEN-91507 (LM-1507) gegebenenfalls in Form ihrer Racemate, Enantiomere oder Diastereomere, gegebenenfalls in Form ihrer pharmakoligisch verträglichen Säureadditionssalze sowie gegebenenfalls in Form ihrer Salze und Derivate, ihrer Solvate und/oder Hydrate.

Unter Säureadditionssalzen mit pharmakologisch verträglichen Säuren zu deren Bildung die LTD4-Antagonisten gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Unter Salzen oder Derivaten zu deren Bildung die LTD4-antagonisten gegebenenfalls in der Lage sind, werden beispielsweise verstanden : Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenylethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluorphenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluorphenyl)amino]-6-(tetrahydropyran-4-yloxy)-7-ethoxy-chinazolin, 4-[(3-Chlor-4-fluorphenyl)amino]-6-((S)-tetrahydrofuran-3-yloxy)-7-hydroxy-chinazolin, 4-[(3-Chlor-4-fluorphenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluorphenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluorphenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-(cis-4-{N-[(morpholin-4-yl)sulfonyl]-N-methyl-amino}-cyclohexan-1-yloxy)-7-methoxy- chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluorphenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluorphenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxyacetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxyl-7-methoxy-chinazolin, 4-[(3-Chlor-4-fluorphenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluorphenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, Cetuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Bevorzugte EGFR-Hemmer sind ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]-amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenylethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenylethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluorphenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(methoxymethyl)carbonyl]-piperidin-4-yl-oxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluorphenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin,
4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluorphenyl)amino]-6-(1-methansulfonyl-piperidin-4-yloxy)-7-ethoxy-chinazolin, 4-[(3-Chlor-4-fluorphenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluorphenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und Cetuximab, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Besonders bevorzugt gelangen im Rahmen der vorliegenden Erfindung diejenigen EGFR-Hemmer zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-{1-[(morpholin-4-yl)carbonyl]-piperidin-4-yl-oxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluorphenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluorphenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluorphenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Erfindungsgemäß besonders bevorzugt sind als EGFR-Hemmer diejenigen Verbindungen, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Ethinyl-phenyl)amino]-6,7-bis-(2-methoxy-ethoxy)-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluorphenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluorphenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluorphenyl)amino]-6-(1-methansulfonyl-piperidin-4-yloxy)-7-methoxy-chinazolin und 4-[(3-Chlor-4-fluor-phenyl)amino]-6-(1-cyano-piperidin-4-yloxy)-7-methoxy-chinazolin gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die EGFR-Hemmer gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopamin-Agonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Eine Bezugnahme auf die vorstehend genannten H1-Antihistaminika schließt im Rahmen der vorliegenden Erfindung eine Bezugnahme auf deren gegebenenfalls existierende pharmakologisch verträgliche Säureadditionssalze ein.

Als PAF-Antagonisten gelangen hierbei vorzugsweise Verbindungen zur Anwendung, die ausgewählt sind aus der Gruppe bestehend aus 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin, 6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin.

Als MRP4-Inhibitoren gelangen hierbei vorzugsweise Verbindungen zur Anwendung, die ausgewählt sind aus der Gruppe bestehend aus *N*-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-S-glutathione, Estradiol 17-beta-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glycocholate, Glycolithocholic acid sulphate, Ibuprofen, Indomethacin, Indoprofen, Ketoprofen, Lithocholic acid sulphate, Methotrexate, MK571 ((*E*)-3-[[[3-[2-(7-Chloro-2-quinolinyl)ethenyl]phenyl]-[[3-dimethylamino)-3-oxopropyl]thio]methyl]thio]-propanoic acid), alpha-Naphthyl-beta-D-glucuronide, Nitrobenzyl mercaptopurine riboside, Probenecid, PSC833, Sildenafil, Sulfinpyrazone, Taurochenodeoxycholate, Taurocholate, Taurodeoxycholate,
Taurolithocholate, Taurolithocholic acid sulphate, Topotecan,
Trequinsin und Zaprinast, Dipyridamol, gegebenenfalls in Form ihrer Racemate, Enantiomere, Diastereomere und ihrer pharmakologisch verträglichen Säure-Additionssalze und Hydrate.

Vorzugsweise bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen PDE4B-Inhibitoren und MRP4-Inhibitoren, wobei die MRP4-Inhibitoren vorzugsweise ausgewählt sind aus der Gruppe bestehend aus *N*-Acetyl-dinitrophenyl-Cysteine, Dehydroepiandrosterone 3-sulphate, Dilazep, Dinitrophenyl-S-glutathione, Estradiol 3,17-disulphate, Flurbiprofen, Glycocholate, Glycolithocholic acid sulphate, Ibuprofen, Indomethacin, Indoprofen, Lithocholic acid sulphate, MK571, PSC833, Sildenafil,
Taurochenodeoxycholate, Taurocholate, Taurolithocholate, Taurolithocholic acid sulphate, Trequinsin und Zaprinast, Dipyridamol, gegebenenfalls in Form ihrer Racemate, Enantiomere, Diastereomere und ihrer pharmakologisch verträglichen Säure-Additionssalze und Hydrate.

Stärker bevorzugt bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen PDE4B-Inhibitoren und MRP4-Inhibitoren, wobei die MRP4-Inhibitoren vorzugsweise ausgewählt sind aus der Gruppe bestehend aus
Dehydroepiandrosterone 3-sulphate, Estradiol 3,17-disulphate, Flurbiprofen, Indomethacin, Indoprofen, MK571, Taurocholate, gegebenenfalls in Form ihrer Racemate, Enantiomere, Diastereomere und ihrer pharmakologisch verträglichen Säure-Additionssalze und Hydrate. Die Trennung von Enantiomeren aus den Racematen kann durch bekannte Verfahren nach dem Stand der Technik durchgeführt werden (z.B. durch Chromatographie an chiralen Phasen etc.).

Mit Säure-Additionssalzen mit pharmakologisch verträglichen Säuren sind z.B. Salze ausgewählt aus der Gruppe bestehend aus Hydrochloriden, Hydrobromiden, Hydroiodiden, Hydrosulphaten, Hydrophosphaten, Hydromethanesulphonaten, Hydronitraten, Hydromaleaten, Hydroacetaten, Hydrobenzoaten, Hydrocitraten, Hydrofumaraten, Hydrotartraten, Hydrooxalaten, Hydrosuccinaten, Hydrobenzoaten and Hydro-p-toluenesulphonaten, vorzugsweise Hydrochloride, Hydrobromide, Hydrosulphate, Hydrophosphate, Hydrofumarate and Hydromethanesulphonate gemeint.

Ein weiterer Gegenstand der Erfindung sind pharmazeutische Zubereitungen, die Dreifachkombinationen von den erfindungsgemäßen PDE4B-Inhibitoren, von MRP4-Inhibitoren und einer weiteren aktiven Substanz wie z.B. einem Anticholinergikum, einem Steroid, einem LTD4-Antagonist oder einem Betamimetikum enthalten, sowie deren Herstellung und deren Verwendung zur Behandlung von Atemwegserkrankungen.

Als iNOS-Inhibitoren gelangen hierbei vorzugsweise Verbindungen zur Anwendung, die ausgewählt sind aus der Gruppe bestehend aus: S-(2-Aminoethyl)isothioharnstoff, Aminoguanidin, 2-Aminomethylpyridin, AMT, L-Canavanin, 2-Iminopiperidin, S-Isopropylisothioharnstoff, S-Methylisothioharnstoff, S-Ethylisothioharnstoff, S-Methyltiocitrullin, S-Ethylthiocitrullin, L-NA (N^{ω}-Nitro-L-arginin), L-NAME (N^{ω}-Nitro-L-argininmethylester), L-NMMA (N^{G}-Monomethyl-L-arginin), L-NIO (N^{ω}-Iminoethyl-L-ornithin), L-NIL (N^{ω}-Iminoethyl-lysin), (S)-6-Acetimidoylamino-2-amino-hexanoic acid (1*H*-tetrazol-5-yl)-amid (SC-51) (J. Med. Chem. 2002, 45, 1686-1689), 1400W, (S)-4-(2-Acetimidoylamino-ethylsulfanyl)-2-amino-buttersäure (GW274150) (Bioorg. Med. Chem. Lett. 2000, 10, 597-600), 2-[2-(4-Methoxy-pyridin-2-yl)-ethyl]-3H-imidazo[4,5-*b*]pyridin (BYK191023) (Mol. Pharmacol. 2006, 69, 328-337), 2-((R)-3-Amino-1-phenyl-propoxy)-4-chlor-5-fluorbenzonitril (WO 01/62704), 2-((1R,3S)-3-Amino-4-hydroxy-1-thiazol-5-yl-butylsulfanyl)-6-trifluoromethyl-nicotinonitril (WO 2004/041794), 2-((1R,3S)-3-Amino-4-hydroxy-1-thiazol-5-yl-butylsulfanyl)-4-chlor-benzonitril (WO 2004/041794), 2-((1R,3S)-3-Amino-4-hydroxy-1-thiazol-5-yl-butylsulfanyl)-5-chlor-benzonitril (WO 2004/041794), (2S,4R)-2-Amino-4-(2-chlor-5-trifluoromethyl-phenylsulfanyl)-4-thiazol-5-yl-butan-1-ol (WO 2004/041794), 2-((1R,3S)-3-Amino-4-hydroxy-l-thiazol-5-yl-butylsulfanyl)-5-chlor-nicotinonitril (WO 2004/041794), 4-((S)-3-Amino-4-hydroxy-1-phenyl-butylsulfanyl)-6-methoxy-nicotinonitril (WO 02/090332), substituierte 3-Phenyl-3,4-dihydro-1-isoquinolinamin wie z.B. AR-C102222 *(*J. Med. Chem. 2003, 46, 913-916), (1S,5S,6R)-7-Chlor-5-methyl-2-aza-bicyclo[4.1.0]hept-2-en-3-ylamin (ONO-1714) (Biochem. Biophys. Res. Commun. 2000, 270, 663-667), (4R,5R)-5-Ethyl-4-methyl-thiazolidin-2-ylideneamin (Bioorg. Med. Chem. 2004, 12, 4101), (4R,5R)-5-Ethyl-4-methyl-selenazolidin-2-ylideneamin (Bioorg. Med. Chem. Lett. 2005, 15, 1361), 4-Aminotetrahydrobiopterin (Curr. Drug Metabol. 2002, 3, 119-121), (E)-3-(4-Chlor-phenyl)-N-(1-{2-oxo-2-[4-(6-trifluormethyl-pyrimidin-4-yloxy)-piperidin-1-yl]-ethylcarbamoyl}-2-pyridin-2-yl-ethyl)-acrylamid (FR260330) (Eur. J. Pharmacol. 2005, 509, 71-76), 3-(2,4-Difluor-phenyl)-6-[2-(4-imidazol-1-ylmethyl-phenoxy)-ethoxy]-2-phenyl-pyridin (PPA250) (J. Pharmacol. Exp. Ther. 2002, 303, 52-57), 3-{[(Benzo[1,3]dioxol-5-ylmethyl)-carbamoyl]-methyl}-4-(2-imidazol-1-yl-pyrimidin-4-yl)-piperazin-1-carbonsäuremethylester (BBS-1) (Drugs Future 2004, 29, 45-52), (R)-1-(2-Imidazol-1-yl-6-methyl-pyrimidin-4-yl)-pyrrolidin-2-carbonsäure (2-benzo[1,3]dioxol-5-yl-ethyl)-amid (BBS-2) *(*Drugs Future 2004, 29, 45-52) und deren pharmazeutischen Salze, Prodrugs oder Solvate.

Als iNOS-Inhibitoren im Rahmen der vorliegenden Erfindung können weiterhin antisense-Oligonucleotide, insbesondere solche antisense-Oligonucleotide, die iNOS-kodierende Nukleinsäuren binden, eingesetzt werden. Z.B. werden in WO 01/52902 antisense-Oligonucleotide, insbesondere antisense-Oligonucleotide, die iNOS kodierende Nukleinsäuren binden, zur Modulierung der Expression von iNOS beschrieben. Solche iNOS-antisense-Oligonucleotide wie insbesondere in WO 01/52902 beschrieben können daher auch aufgrund ihrer ähnlichen Wirkung wie die iNOS-Inhibitoren mit den PDE4-Inhibitoren der vorliegenden Erfindung kombiniert werden.

Als SYK-Inhibitoren gelangen hierbei vorzugsweise Verbindungen zur Anwendung, die ausgewählt sind aus der Gruppe bestehend aus: 2-[(2-aminoethyl)amino]-4-[(3-bromophenyl)amino]-5-Pyrimidinecarboxamide;
2-[[7-(3,4-dimethoxyphenyl)imidazo[1,2-c]pyrimidin-5-yl]amino]-3-Pyridinecarboxamide;
6-[[5-fluoro-2-[3,4,5-trimethoxyphenyl)amino]-4-pyrimidinyl]amino]-2,2-dimethyl-2H-Pyrido[3,2-b]-1,4-oxazin-3(4H)-one;
N-[3-bromo-7-(4-methoxyphenyl)-1,6-naphthyridin-5-yl]-1,3-Propanediamine 7-(4-methoxyphenyl)-N-methyl-1,6-Naphthyridin-5-amine;
N-[7-(4-methoxyphenyl)-1,6-naphthyridin-5-yl]-1,3-Propanediamine;
N-[7-(2-thienyl)-1,6-naphthyridin-5-yl-1,3-Propanediamine;
N-[7-[4-(dimethylamino)phenyl]-1,6-naphthyridin-5-yl]-1,2-Ethanediamine;
N-[7-(4-methoxyphenyl)-2-(trifluoromethyl)-1,6-naphthyridin-5-yl]-1,3-Propanediamine;
N-[7-(4-methoxyphenyl)-3-phenyl-1,6-naphthyridin-5-yl]-1,3-Propanediamine;
N-(7-phenyl-1,6-naphthyridin-5-yl)-1,3-Propanediamine;
N-[7-(3-fluorophenyl)-1,6-naphthyridin-5-yl]-1,3-Propanediamine;
N-[7-(3-chlorophenyl)-1,6-naphthyridin-5-yl]-1,3-Propanediamine;
N-[7-[3-(trifluoromethoxy)phenyl]-1,6-naphthyridin-5yl]-1,3-Propanediamine;
N-[7-(4-fluorophenyl)-1,6-naphthyridin-5-yl]-1,3-Propanediamine;
N-[7-(4-fluorophenyl)-1,6-naphthyridin-5-yl]-1,3-Propanediamine;
N-[7-(4-chlorophenyl)-1,6-naphthyridin-5-yl]-1,3-Propanediamine;
N-[7-(4'-methyl[1,1'-biphenyl]-4-yl)-1,6-naphthyridin-1,3-Propanediamine;
N-[7-[4-(dimethylamino)phenyl]-1,6-naphthyridin-5-yl]-1,3-Propanediamine;
N-[7-[4-(diethylamino)phenyl]-1,6-naphthyridin-5-yl]-1,3-Propanediamine;
N-[7-[4-(4-morpholinyl)phenyl]-1,6-naphthyridin-5-yl]-1,3-Propanediamine;
N-[7-[4-[[2-(dimethylamino)ethyl]methylamino]phenyl]-1,6-naphthyridin-5-yl]-1,3-Propanediamine;
N-[7-(4-bromophenyl)-1,6-naphthyridin-5-yl]-1,3-Propanediamine;
N-[7-(4-methylphenyl)-1,6-naphthyridin-5-yl]-1,3-Propanediamine;
N-[7-[4-(methylthio)phenyl]-1,6-naphthyridin-5-yl]-1,3-Propanediamine;
N-[7-[4-(1-methylethyl)phenyl]-1,6-naphthyridin-5-yl]-1,3-Propanediamine;
7-[4-(dimethylamino)phenyl]-N-methyl-1,6-Naphthyridin-5-amine;
7-[4-(dimethylamino)phenyl]-N,N-dimethyl-1,6-Naphthyridin-5-amine;
N-[7-[4-(dimethylamino)phenyl]-1,6-naphthyridin-5-yl]-1,4-Butanediamine;
N-[7-[4-(dimethylamino)phenyl]-1,6-naphthyridin-5-yl]-1,5-Pentanediamine;
3-[[7-[4-(dimethylamino)phenyl]-1,6-naphthyridin-5-yl]oxy]-1-Propanol;
4-[5-(4-aminobutoxy)-1,6-naphthyridin-7-yl]-N,N-dimethyl-Benzenamine;
4-[[7-[4-(dimethylamino)phenyl]-1,6-naphthyridin-5-yl]amino]-1-Butanol;
N-[7-[4-(dimethylamino)phenyl]-1,6-naphthyridin-5-yl]-N-methyl-1,3-Propanediamine;
N-[7-[4-(dimethylamino)phenyl]-1,6-naphthyridin-5-yl]-N'-methyl-1,3-Propanediamine;
N-[7-[4-(dimethylamino)phenyl]-1,6-naphthyridin-5-yl]-N,N'-dimethyl-1,3-Propanediamine;
1-amino-3-[[7-[4-(dimethylamino)phenyl]-1,6-naphthyridin-5-yl]amino]-2-Propanol;
N-[7-[4-(dimethylamino)phenyl]-1,6-naphthyridin-5-yl]-2,2-dimethyl-1,3-Propanediamine;
7-[4-(dimethylamino)phenyl]-N-(3-pyridinylmethyl)-1,6-Naphthyridin-5-amine;
N-[(2-aminophenyl)methyl]-7-[4-(dimethylamino)phenyl]-1,6-Naphthyridin-5-amine;
N-[7-[6-(dimethylamino)[1,1'-biphenyl]-3-yl]-1,6-naphthyridin-5-yl]-1,3-Propanediamine, ;
N-[7-[3-chloro-4-(diethylamino)phenyl]-1,6-naphthyridin-5-yl]-1,3-Propanediamine;
N-[7-[4-(dimethylamino)-3-methoxyphenyl]-1,6-naphthyridin-5-yl]-1,3-Propanediamine;
N-[7-[4-(diethylamino)phenyl]-3-methyl-1,6-naphthyridin-5-yl]-1,3-Propanediamine;
N-[7-(3'-fluoro[1,1'-biphenyl]-3-yl)-1,6-naphthyridin-5-yl]-1,2-Ethanediamine,
N-[7-(4-methoxyphenyl)-1,6-naphthyridin-5-yl]-1,6-Naphthyridine-1,3-Propanediamine;
N,N'-bis(3-aminopropyl)-7-(4-methoxyphenyl)-2,5-diamine;
N-[7-(4-methoxyphenyl)-2-(phenylmethoxy)-1,6-naphthyridin-5-yl]-1,6-Naphthyridine-1,3-Propanediamine;
N5-(3-aminopropyl)-7-(4-methoxyphenyl)-N2-(phenylmethyl)-2,5-diamine;
N-[7-(2-naphthalenyl)-1,6-naphthyridin-5-yl]-1,3-Propanediamine;
N-[7-(2'-fluoro[1,1'-biphenyl]-4-yl)-1,6-naphthyridin-5-yl]-1,3-Propanediamine;
N-[7-(3,4,5-trimethoxyphenyl)-1,6-naphthyridin-5-yl]-1,3-Propanediamine;
N-[7-(3,4-dimethylphenyl)-1,6-naphthyridin-5-yl]-1,3-Propanediamine;
1-amino-3-[[7-(2-naphthalenyl)-1,6-naphthyridin-5-yl]amino]-2-Propanol;
1-amino-3-[[7-(2'-fluoro[1,1'-biphenyl]-4-yl)-1,6-naphthyridin-5-yl]amino]-2-Propanol;
1-amino-3-[[7-(4'-methoxy[1,1'-biphenyl]-4-yl)-1,6-naphthyridin-5-yl]amino]-2-Propanol;
1-amino-3-[[7-(3,4,5-trimethoxyphenyl)-1,6-naphthyridin-5-yl]amino]-2-Propanol;
1-amino-3-[[7-(4-bromophenyl)-1,6-naphthyridin-5-yl]amino]-2-Propanol;
N-[7-(4'-methoxy[1,1'-biphenyl]-4-yl)-1,6-naphthyridin-5-yl]-2,2-dimethyl-1,3-Propanediamine;
1-[[7-[4-(dimethylamino)phenyl]-1,6-naphthyridin-5-yl]amino]-2-Propanol;
2-[[2-[[7-[4-(dimethylamino)phenyl]-1,6-naphthyridin-5-yl]amino]ethyl]thio]-Ethanol;
7-[4-(dimethylamino)phenyl]-N-(3-methyl-5-isoxazolyl)-1,6-Naphthyridin-5-amine;
7-[4-(dimethylamino)phenyl]-N-4-pyrimidinyl-1,6-Naphthyridin-5-amine;
N-[7-[4-(dimethylamino)phenyl]-1,6-naphthyridin-5-yl]-1,3-Cyclohexanediamine;
N,N-dimethyl-4-[5-(1-piperazinyl)-1,6-naphthyridin-7-yl]-Benzenamine;
4-[5-(2-methoxyethoxy)-1,6-naphthyridin-7-yl]-N,N-dimethyl-Benzenamine;
1-[7-[4-(dimethylamino)phenyl]-1,6-naphthyridin-5-yl]-4-Piperidinol;
1-[7-[4-(dimethylamino)phenyl]-1,6-naphthyridin-5-yl]-3-Pyrrolidinol;
7-[4-(dimethylamino)phenyl]-N-(2-furanylmethyl)-1,6-Naphthyridin-5-amine;
7-[4-(dimethylamino)phenyl]-N-[3-(1H-imidazol-1-yl)propyl]-1,6-Naphthyridin-5-amine;
1-[7-[4-(dimethylamino)phenyl]-1,6-naphthyridin-5-yl]-4-Piperidinecarboxamide;
1-[3-[[7-[4-(dimethylamino)phenyl]-1,6-naphthyridin-5-yl]amino]propyl]-2-Pyrrolidinone;
N-[3'-[5-[(3-aminopropyl)amino]-1,6-naphthyridin-7-yl][1,1'-biphenyl]-3-yl]-Acetamide;
N-[7-(4'-fluoro[1,1'-biphenyl]-4-yl)-1,6-naphthyridin-5-yl]-1,3-Propanediamine;
N-[4'-[5-[(3-aminopropyl)amino]-1,6-naphthyrid in-7-yl][1,1'-biphenyl]-3-yl]-Acetamide;
N-[7-[4-(1,3-benzodioxol-5-yl)phenyl]-1,6-naphthyridin-5-yl]-1,3-Propanediamine;
N-[7-[4-(2-thienyl)phenyl]-1,6-naphthyridin-5-yl]-1,3-Propanediamine;
N-[7-[4-fluoro-3-(trifluoromethyl)phenyl]-1,6-naphthyridin-5-yl]-1,3-Propanediamine;
N-[7-[4-(3-pyridinyl)phenyl]-1,6-naphthyridin-5-yl]-1,3-Propanediamine;
N-[7-(1,3-benzodioxol-5-yl)-1,6-naphthyridin-5-yl]-1,3-Propanediamine;
N-[7-(6-methoxy-2-naphthalenyl)-1,6-naphthyridin-5-yl]-1,3-Propanediamine;
7-[4-(dimethylamino)phenyl]-N-(4-pyridinylmethyl)-1,6-Naphthyridin-5-amine;
3-[[7-[4-(dimethylamino)phenyl]-1,6-naphthyridin-5-yl]methylamino]-Propanenitrile;
7-[4-(dimethylamino)phenyl]-N-[1-(phenylmethyl)-4-piperidinyl]-1,6-Naphthyridin-5-amine;
N-[7-[4-(dimethylamino)phenyl]-1,6-naphthyridin-5-yl]-1,2-Cyclohexanediamine, (1 R,2S)-rel-.
N-[7-[4-(dimethylamino)phenyl]-1,6-naphthyridin-5-yl]-1,2-Benzenedimethanamine;
N-[7-[4-(diethylamino)phenyl]-1,6-naphthyridin-5-yl]-1,4-Butanediamine;
N-[7-[3',5'-bis(trifluoromethyl)[1,1'-biphenyl]-4-yl]-1,6-naphthyridin-5-yl]-,3-Propanediamine;
N-[7-(3'-methoxy[1,1'-biphenyl]-4-yl)-1,6-naphthyridin-5-yl]-1,3-Propanediamine;
N-[7-(3'-fluoro[1,1'-biphenyl]-4-yl)-1,6-naphthyridin-5-yl]-1,3-Propanediamine;
4-[[7-[4-(dimethylamino)phenyl]-1,6-naphthyridin-5-yl]oxy]-1-Butanol;
N-[7-[4-(dimethylamino)phenyl]-1,6-naphthyridin-5-yl]-1,4-Cyclohexanediamine;
7-[4-(dimethylamino)phenyl]-N-(2,2,6,6-tetramethyl-4-piperidinyl)-1,6-Naphthyridin-5-amine;
N-[7-[3-bromo-4-(dimethylamino)phenyl]-1,6-naphthyridin-5-yl]-1,3-Propanediamine;
N-[7-(1-methyl-1H-indol-5-yl)-1,6-naphthyridin-5-yl]-1,3-Propanediamine;
N-[7-[3-(trifluoromethyl)phenyl]-1,6-naphthyridin-5-yl]-1,3-Propanediamine;
N-[7-[4-(trifluoromethyl)phenyl]-1,6-naphthyridin-5-yl]-1,3-Propanediamine;
N-[7-(3-bromo-4-methoxyphenyl)-1,6-naphthyridin-5-yl]-1,3-Propanediamine;
N-[7-[4-[[3-(dimethylamino)propyl]methylamino]phenyl]-1,6-naphthyridin-5-yl]-1,4-Cyclohexanediamine;
N-[7-[4-[[2-(dimethylamino)ethyl]methylamino]phenyl]-1,6-naphthyridin-5-yl]-1,4-Cyclohexanediamine;
N-[7-[4-(dimethylamino)-3-methoxyphenyl]-1,6-naphthyridin-5-yl]-1,4-Cyclohexanediamine;
N-[7-[4-(4-morpholinyl)phenyl]-1,6-naphthyridin-5-yl]-1,4-Cyclohexanediamine;
N-[7-[3-bromo-4-(4-morpholinyl)phenyl]-1,6-naphthyridin-5-yl]-1,4-Cyclohexanediamine;
4-[[7-[4-[[2-(dimethylamino)ethyl]methylamino]phenyl]-1,6-naphthyridin-5-yl]oxy]-Cyclohexanol;
N-[7-[3-bromo-4-(4-morpholinyl)phenyl]-1,6-naphthyridin-5-yl]-1,3-Propanediamine;
N,N-dimethyl-4-[5-(4-methyl-1-piperazinyl)-1,6-naphthyridin-7-yl]-Benzenamine;
4-[[7-[4-[[3-(dimethylamino)propyl]methylamino]phenyl]-1,6-naphthyridin-5-yl]oxy]-Cyclohexanol;
N-[7-[4-[[2-(dimethylamino)ethyl]methylamino]phenyl]-1,6-naphthyridin-5-yl]-1,4-Butanediamine;
[3-[[5-[(3-aminopropyl)amino]-7-(4-methoxyphenyl)-1,6-naphthyridin-2-yl]amino]propyl]-Carbamic acid-1,1-dimethylethyl ester.

### DARREICHUNGSFORMEN

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0, 1 bis 90 Gew.-%, bevorzugt 0, 5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel **A** gemäß der obigen bevorzugten Ausführungsformen.

Besonders bevorzugt ist es, wenn die Verbindungen der Formel **A** oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel **A** inhalativ verabreicht werden, besonders bevorzugt ist es, wenn die Verabreichung ein oder zweimal täglich erfolgt. Hierzu müssen die Verbindungen der Formel **A** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel **A** im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronisieren sowie durch abschließendes Mischen der Bestandteile sind aus dem Stand der Technik bekannt.

### Treibgashaltige Inhalationsaerosole

Die im Rahmen der erfindungsgemäßen Verwendung einsetzbaren treibgashaltigen Inhalationsaerosole können die Verbindungen nach Formel **A** im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1, 1, 1, 2-Tetrafluorethan), TG227 (1, 1, 1, 2, 3, 3, 3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindungsgemäße Verwendung von Verbindungen der Formel **A** erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, Dihydrothienopyrimidin und ein oder mehrere Kombinationspartner ausgewählt aus der oben beschriebenen Gruppe, bereit gestellt.

## Patentansprüche

1. Verbindung der Formel **A**, 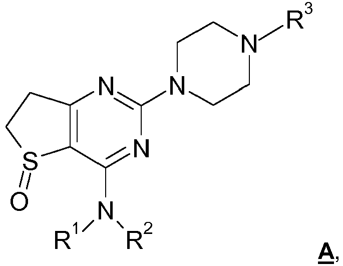 wobei
**R¹** H ist,
**R²** ausgewählt ist aus der Gruppe bestehend aus 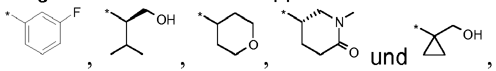 und
**R³** ein bicyclischer 9- bis 11-gliedriger Heterocyclus ausgewählt aus der Gruppe bestehend aus Benzoxazol, Benzodioxol, Dihydrobenzodioxin, Benzodioxin, Benzisoxazol, Benzothiazol, Benzisothiazol, Thienopyrimidin, Furopyrimidin, Thienopyridin, Furopyridin, Indol, Isoindol, Chinoxalin, Naphthyridin, Pyridopyrazin, Pyridopyrimidin, Chinolin, Isochinolin, Benzoimidazol, 6,7,8,9-Tetrahydro-5H-pyrazino[2,3-d]azepin, Benzothiophen, Benzofuran, Chinazolin, Indazol, Isobenzofuran und Pteridin ist,
der gegebenenfalls durch einen oder mehrere Reste ausgewählt aus der Gruppe bestehend aus
F, Cl, Br, CF₃, CHF₂, CH₂F, CN, OH, -Methyl, Ethyl, Propyl, Isopropyl, -O-Methyl und O-Ethyl, substituiert sein kann,
die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl, - COO(CH₃), -O-Methyl und -O-Ethyl substituiert sein können,
sowie pharmakologisch verträgliche Salze, Diastereomere, Enantiomere, Racemate, Hydrate und Solvate davon.

2. Verbindung der Formel **A**
nach Anspruch 1, wobei
**R³** ausgewählt ist aus der Gruppe bestehend aus 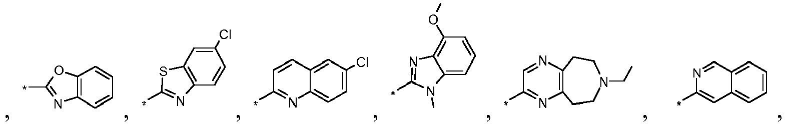 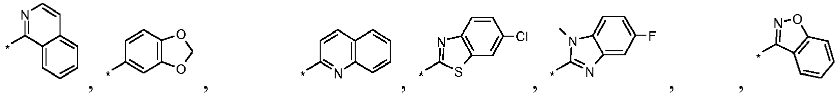 und 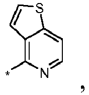
sowie pharmakologisch verträgliche Salze, Diastereomere, Enantiomere, Racemate, Hydrate und Solvate davon.

3. Verbindungen nach einem der Ansprüche 1 oder 2 als Arzneimittel.

4. Verbindungen nach einem der Ansprüche 1 oder 2 zur Verwendung in einem Verfahren zur Behandlung von Atemwegs- oder gastrointestinalen Beschwerden oder Erkrankungen, wie auch entzündliche Erkrankungen der Gelenke, der Haut, der Augen oder des Gastrointestinaltraktes, Krebserkrankungen, sowie von Erkrankungen des periphären oder zentralen Nervensystems.

5. Verbindungen nach einem der Ansprüche 1 oder 2 zur Verwendung in einem Verfahren zur Vorbeugung und Behandlung von Atemwegs- oder Lungenerkrankungen, die mit einer erhöhten Schleimproduktion, Entzündungen und / oder obstruktiven Erkrankungen der Atemwege einher gehen.

6. Verbindungen nach einem der Ansprüche 1 oder 2 zur Verwendung in einem Verfahren zur Behandlung von entzündlichen und obstruktiven Erkrankungen ausgewählt aus der Gruppe bestehend aus COPD, chronischer Sinusitis, Asthma, Morbus Crohn, idiopathischer Lungenfibrose und Colitis ulcerosa.

7. Verbindungen nach einem der Ansprüche 1 oder 2 zur Verwendung in einem Verfahren zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

8. Pharmazeutische Formulierungen **gekennzeichnet durch** den Gehalt an einer oder mehreren Verbindungen der Formel **1** nach einem der Ansprüche 1 oder 2.

9. Pharmazeutische Formulierungen **gekennzeichnet durch** den Gehalt an einer oder mehreren Verbindungen der Formel **1** nach einem der Ansprüche 1 oder 2 in Kombination mit einem oder mehreren Wirkstoffen ausgewählt aus der Gruppe bestehend aus Betamimetika, Corticosteroiden, weiteren PDE4-Inhibitoren, EGFR-Hemmern und LTD4-Antagonisten, CCR3-Inhibitoren, iNOS-Inhibitoren, MRP4-Inhibitoren und SYK-Inhibitoren.

## Claims

1. Compound of formula **A** 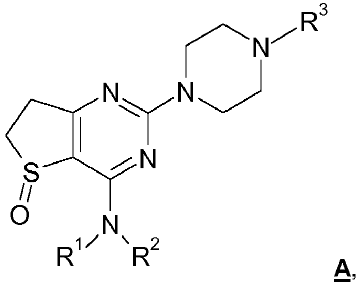 wherein
**R¹** is H,
**R²** is selected from among 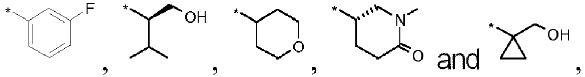
and
**R³** is a bicyclic 9- to 11-membered heterocycle selected from among benzoxazole, benzodioxole, dihydrobenzodioxin, benzodioxin, benzisoxazole, benzothiazole, benzisothiazole, thienopyrimidine, furopyrimidine, thienopyridine, furopyridine, indole, isoindole, quinoxaline, naphthyridine, pyridopyrazine, pyridopyrimidine, quinoline, isoquinoline, benzimidazole, 6,7,8,9-tetrahydro-5H-pyrazino[2,3-d]azepine, benzothiophene, benzofuran, quinazoline, indazole, isobenzofuran and pteridine, which may optionally be substituted by one or more groups selected from among F, Cl, Br, CF₃, CHF₂, CH₂F, CN, OH, -methyl, ethyl, propyl, isopropyl, -O-methyl and O-ethyl,
which may in turn optionally be substituted by one or more groups selected from among OH, F, Cl, Br, CF₃, CHF₂, CH₂F, methyl, ethyl, propyl, isopropyl, phenyl, -COO(CH₃), -O-methyl and -O-ethyl,
as well as pharmacologically acceptable salts, diastereomers, enantiomers, racemates, hydrates and solvates thereof.

2. Compound of formula **A** according to Claim 1, wherein
**R³** is selected from among 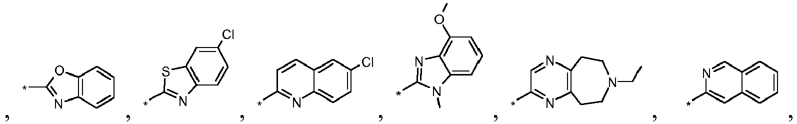 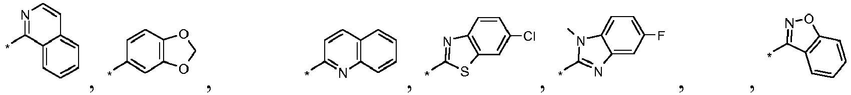 and 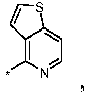
as well as pharmacologically acceptable salts, diastereomers, enantiomers, racemates, hydrates and solvates thereof.

3. Compounds according to either Claim 1 or Claim 2 as medicaments.

4. Compounds according to either Claim 1 or Claim 2 for use in a method for the treatment of respiratory or gastrointestinal diseases or complaints, as well as inflammatory diseases of the joints, skin, eyes or the gastrointestinal tract, cancers, and diseases of the peripheral or central nervous system.

5. Compounds according to either Claim 1 or Claim 2 for use in a method for the prevention and treatment of respiratory or pulmonary diseases which are accompanied by increased mucus production, inflammations and/or obstructive diseases of the respiratory tract.

6. Compounds according to either Claim 1 or Claim 2 for use in a method for the treatment of inflammatory and obstructive diseases selected from among COPD, chronic sinusitis, asthma, Crohn's disease, idiopathic pulmonary fibrosis and ulcerative colitis.

7. Compounds according to either Claim 1 or Claim 2 for use in a method for the prevention and treatment of diseases of the peripheral or central nervous system such as depression, bipolar or manic depression, acute and chronic anxiety states, schizophrenia, Alzheimer's disease, Parkinson's disease, acute and chronic multiple sclerosis or acute and chronic pain and brain damage caused by stroke, hypoxia or cranio-cerebral trauma.

8. Pharmaceutical formulations, **characterised in that** they contain one or more compounds of formula **1** according to either Claim 1 or Claim 2.

9. Pharmaceutical formulations, **characterised in that** they contain one or more compounds of formula **1** according to either Claim 1 or Claim 2 in combination with one or more active substances selected from among betamimetics, corticosteroids, other PDE4-inhibitors, EGFR-inhibitors and LTD4-antagonists, CCR3-inhibitors, iNOS-inhibitors, MRP4-inhibitors and SYK-inhibitors.

## Revendications

1. Composé de formule **A**, 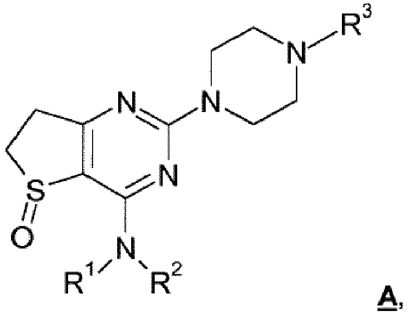 dans laquelle
**R¹** est H,
**R²** est choisi dans le groupe constitué de 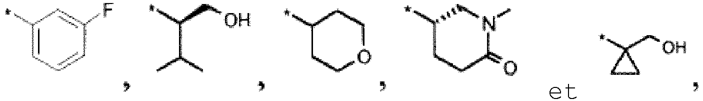
et
**R³** est un hétérocycle bicyclique de 9 à 11 chaînons choisi dans le groupe constitué de benzoxazol, benzodioxol, dihydrobenzodioxine, benzodioxine, benzisoxazol, benzothiazol, benzisothiazol, thiénopyrimidine, furopyrimidine, thiénopyridine, furopyridine, indole, iso-indole, quinoxaline, naphthydrine, pyridopyrazine, pyridopyrimidine, quinoline, isoquinoline, benzo-imidazol, 6,7,8,9-tétrahydro-5H-pyrazino[2,3-d]azépine,
benzothiophène, benzofurane, quinazoline, indazole, isobenzofurane et ptéridine,
qui peut être éventuellement substitué par un ou plusieurs radicaux choisis dans le groupe constitué de
F, Cl, Br, CF₃, CHF₂, CH₂F, CN, OH, -méthyle, éthyle, propyle, isopropyle, -0-méthyle et O-éthyle,
qui peuvent eux-mêmes être éventuellement substitués par un ou plusieurs radicaux choisis dans le groupe OH, F, Cl, Br, CF₃, CHF₂, CH₂F, méthyle, éthyle, propyle, isopropyle, phényle, - COO(CH₃), -0-méthyle et -O-éthyle,
ainsi que leurs sels, diastéréomères, énantiomères, racémates, hydrates et solvates pharmacologiquement acceptables.

2. Composé de formule **A**
selon la revendication 1, dans laquelle
**R³** est choisi dans le groupe constitué de 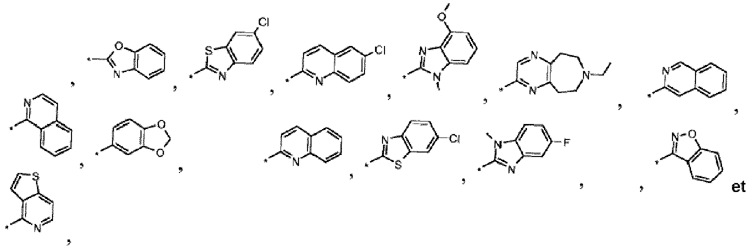
ainsi que leurs sels, diastéréomères, énantiomères, racémates, hydrates et solvates pharmacologiquement acceptables.

3. Composés selon l'une des revendications 1 ou 2 en tant que médicaments.

4. Composés selon l'une des revendications 1 ou 2 pour utilisation dans un procédé pour le traitement des troubles ou maladies des voies respiratoires ou gastro-intestinales en des maladies, comme également des maladies inflammatoires des articulations, de la peau, des yeux ou des voies gastro-intestinales, des cancers ainsi que des maladies du système nerveux périphérique ou central.

5. Composés selon l'une des revendications 1 ou 2, pour utilisation dans un procédé de prévention et de traitement des maladies des voies respiratoires ou pulmonaires qui s'accompagnent d'une production accrue de mucus, d'inflammations et/ou de maladies obstructives des voies respiratoires.

6. Composés selon l'une des revendications 1 ou 2 pour utilisation dans un procédé pour le traitement de maladies inflammatoires et obstructives choisies dans le groupe constitué de la BPCO, la sinusite chronique, l'asthme, la maladie de Crohn, la fibrose pulmonaire idiopathique et la rectocolite hémorragique.

7. Composés selon l'une des revendications 1 ou 2, pour l'utilisation dans un procédé pour la prévention et le traitement de maladies du système nerveux périphérique ou central telles que la dépression, la dépression bipolaire ou maniaque, les états d'angoisse aiguë ou chronique, la schizophrénie, la maladie d'Alzheimer, la maladie de Parkinson, la sclérose en plaque aiguë et chronique ou les états douloureux aigus et chroniques ainsi que les lésions du cerveau provoquées par un AVC, une hypoxie, ou un traumatisme crânien.

8. Formulations pharmaceutiques **caractérisées par** la teneur en un ou plusieurs composés de formule **1** selon l'une des revendications 1 ou 2.

9. Formulations pharmaceutiques **caractérisées par** la teneur en un ou plusieurs composés de formule **1** selon l'une des revendications 1 ou 2 en combinaison avec un ou plusieurs principes actifs choisis dans le groupe constitué des bêtamimétiques, corticoïdes, d'autres inhibiteurs de PDE4, des inhibiteurs de l'EGFR et des antagonistes de LTD4, des inhibiteurs de CCR3, des inhibiteurs de l'iNOS, des inhibiteurs de MRP4 et des inhibiteurs de SYK.
